# EUROPEAN PATENT APPLICATION

(11) **EP 4 410 217 A1**
(43) Date of publication of application: **07.08.2024**
(21) Application number: 24155547.3
(22) Date of filing: 02.02.2024
(51) Int. Cl.: A61B 8/06, A61B 5/026, A61B 5/1455, A61B 5/00, A61B 8/08, A61B 8/00, A61H 31/00

(54) **NONINVASIVELY DETECTING BLOOD FLOW TO THE BRAIN**

(30) Priority: 03.02.2023 US 202363443301 P
(71) Applicant: Stryker Corporation, Kalamazoo, MI 49002 (US)
(72) Inventor: SIEDENBURG, Clinton T., Kalamazoo, 49002 (US); CHAPMAN, Fred W., Kalamazoo, 49002 (US); PIRAINO, Daniel W., Kalamazoo, 49002 (US); TAYLOR, Tyson G., Kalamazoo, 49002 (US)
(74) Representative: V.O.

(57) **Abstract**

Implementations for detecting blood flow through one or more blood vessels of a patient may include a flow monitor suited to detect blood flow to the brain during emergency situations. The flow monitor may be disposed on the skin of a patient and may emit an incident beam (e.g., ultrasound and/or infrared light) at an angle that is non-parallel to the blood vessel(s). The flow monitor may determine the velocity of blood flowing through the blood vessel(s) by detecting a Doppler shift in a reflection of the incident beam from the blood.

## Description

### CROSS-REFERENCE TO RELATED APPLICATION

This application claims priority to U.S. Provisional App. No. 63/443,301, which is titled "Blood Flow Monitors," was filed on February 3, 2023, and is hereby incorporated by reference in its entirety.

### BACKGROUND

The velocity of blood flowing through a patient can be detected noninvasively, for instance, using Doppler ultrasound. An ultrasound beam can be transmitted through the patient's skin and toward blood flowing through the blood vessel. When the ultrasound beam is reflected from cells in the moving blood, the reflection of the ultrasound beam has a phase or frequency shift with respect to the incident beam. The difference between the frequency of the incident beam and the frequency of the reflection of the ultrasound beam is the Doppler shift for continuous wave Doppler. The difference between the phase shift of the reflections of the ultrasound beam at a particular point over time is the Doppler shift for pulse wave Doppler. The Doppler shift is dependent on the velocity of the moving blood. By detecting the reflection of the ultrasound beam, the velocity of the blood can be determined. Therefore, blood velocity can be detected using ultrasound.

### SUMMARY

According to an aspect is provided an external flow monitor, comprising: an emitter configured to output an incident beam toward a brain of an, e.g. adult, subject; a receiver configured to detect a reflection of the incident beam from blood vessels in the brain; a processor configured to: determine, by analyzing data indicative of the reflection of the incident beam, a magnitude of blood flowing through the brain during an event, the event comprising a cardiac cycle or chest compression; and output a signal indicative of the magnitude of blood flowing in the brain.

Optionally, the incident beam comprises ultrasound having a frequency in a range of 100 kHz to 2 MHz.

Optionally, the incident beam comprises infrared light.

Optionally, the emitter is configured to output the incident beam through an orbital cavity, a temple, or an ear canal of the subject. The blood vessels can e.g. be located in a circle of Willis of the subject.

Optionally, the processor is configured to determine the magnitude of the blood flowing in the brain by comparing a frequency of the reflection of the incident beam to a frequency of the incident beam.

Optionally, the receiver is configured to detect a reflection of the incident beam from a contrast agent in the blood in the blood vessel.

Optionally, the external flow monitor comprises a transceiver, and the processor is configured to output the signal by causing the transceiver to transmit the signal to a medical device or a mobile device.

Optionally, the external flow monitor comprises an output device, and the processor is configured to output the signal by causing the output device to output the signal to a user.

Optionally, the processor is configured to determine a volume of the blood flowing through the brain by integrating a velocity of the blood flowing in the blood vessel with respect to time.

Optionally, the processor is configured to determine that the volume of the blood flowing through the brain is below a threshold. Optionally, the signal indicative of the magnitude of blood flowing in the blood vessel comprises an instruction, such as to a mechanical chest compression device, to initiate or change additional chest compressions administered to the subject. Optionally, the signal indicative of the magnitude of blood flowing in the blood vessel comprises an instruction, such as to a mechanical chest compression device, to change a position, frequency, timing, or depth of the chest compressions.

Optionally, the external flow monitor comprises a housing. The emitter can be disposed in the housing. The receiver can be disposed in the housing.

Optionally, the external flow monitor comprises a strap coupled to the housing, e.g. configured to hold the external flow monitor on an external surface of a head of the subject.

According to an aspect is provided a resuscitation system, comprising a mechanical chest compression device configured to administer chest compressions to a subject, and the external flow monitor as described hereinabove. The processor can be configured to, on the basis of the determined magnitude of blood flowing in the blood vessels in the brain of the subject, cause the transceiver to transmit, to the mechanical chest compression device, a signal instructing the mechanical chest compression device to initiate chest compressions and/or to change a position, frequency, timing, or depth of the chest compressions.

The processor can be configured to determine that the magnitude of blood flowing in the blood vessels in the brain is below a threshold; and in response to determining that the magnitude of blood flowing in the blood vessels in the brain is below the threshold, cause the transceiver to transmit, to the mechanical chest compression device, a signal instructing the mechanical chest compression device to change a position, frequency, timing, or depth of the chest compressions.

According to an aspect is provided a resuscitation system, comprising a mechanical chest compression device configured to administer chest compressions to a subject, and an external flow monitor. The external flow monitor comprises a housing. The external flow monitor can comprise a strap coupled to the housing, and e.g. configured to hold the external flow monitor on an external surface of, e.g. a head of, the subject. The external flow monitor can comprise an emitter, e.g. disposed in the housing, configured to output an ultrasound beam toward the subject. The external flow monitor can comprise a receiver, e.g. disposed in the housing, configured to detect a reflection of the ultrasound beam from blood vessels in a brain of the subject. The external flow monitor can comprise a transceiver. The external flow monitor can comprise a processor. The processor can be configured to determine a magnitude of blood flowing in the blood vessels in the brain of the subject by analyzing the reflection of the ultrasound beam from the blood vessel in the brain of the subject. The processor can be configured to determine that the magnitude of blood flowing in the blood vessels in the brain is below a threshold. The processor can be configured to, in response to determining that the magnitude of blood flowing in the blood vessels in the brain is below the threshold, cause the transceiver to transmit, to the mechanical chest compression device, a signal instructing the mechanical chest compression device to change a position, frequency, timing, or depth of the chest compressions.

Optionally, the emitter is configured to output the ultrasound beam through an orbital cavity, a temple, or an ear canal of the subject. The blood vessels can be located in a circle of Willis of the subject.

Optionally, the ultrasound beam has a frequency in a range of 100 kHz to 2 MHz.

According to an aspect is provided a method, comprising: transmitting an incident beam toward a brain of a subject; detecting a reflection of the incident beam from blood in blood vessels in the brain of the subject; estimating a magnitude of blood flowing through the brain by analyzing data indicative of the reflection of the incident beam; and outputting a signal indicative of the magnitude of blood flowing through the brain.

Optionally, the incident beam comprises ultrasound having a frequency in a range of 100 kHz to 2 MHz.

Optionally, the incident beam comprises infrared light.

Optionally, transmitting the incident beam toward the blood vessel in the brain of the subject comprises transmitting the incident beam through an orbital cavity, a temple, or an ear canal of the subject.

Optionally, determining the magnitude of the blood flowing through the brain comprises comparing a frequency of the reflection of the incident beam to a frequency of the incident beam.

Optionally, outputting the signal indicative of the magnitude of blood flowing through the brain comprises transmitting the signal to a medical device or to a mobile device.

Optionally, outputting the signal indicative of the magnitude of blood flowing through the brain comprises causing an output device to output the signal to a user.

Optionally, the method comprises determining that the magnitude of the blood flowing through the brain is below a threshold. Optionally, the signal indicative of the magnitude of blood flowing through the brain comprises an instruction to initiate or change chest compressions administered to the subject.

It will be appreciated that any of the aspects, features and options described herein can be combined. It will particularly be appreciated that any of the aspects, features and options described in view of the external flow monitor apply equally to the resuscitation system and method, and vice versa.

### BRIEF DESCRIPTION OF THE DRAWINGS

FIG. 1 illustrates an example environment for monitoring an amount of blood flowing through a brain by monitoring blood vessels supplying blood to the brain. FIG. 1 includes a subject who is experiencing a medical emergency, a rescuer, and a flow monitor.
FIG. 2 illustrates an environment for monitoring blood flow in a brain of a subject 202 by monitoring blood vessels in the brain through an opening in a skull of the subject.
FIG. 3 illustrates the structure of an example flow monitor.
FIG. 4A illustrates an example method for determining a net flow volume of blood though a brain of a subject.
FIG. 4B Illustrates an example method for determining a magnitude of blood though a brain of a subject.
FIG. 5 illustrates an example environment for monitoring blood flow through one or more blood vessels of a subject.
FIG. 6 illustrates various placements of a flow monitor on the body of a subject.
FIG. 7 illustrates an example of an external defibrillator configured to administer an electrical shock to a subject and receive an output from a flow monitor indicating the status of blood flow through the brain of the subject.
FIG. 8 illustrates a chest compression device configured to administer a chest compression to a subject and receive an output from a flow monitor indicating the status of blood flow through the brain of the subject.
FIG. 9 illustrates a flow monitor configured to monitor blood flow through one or more blood vessels of a subject.

### DETAILED DESCRIPTION

When a patient is in cardiac arrest, the heart of the patient is unable to pump blood effectively to the body of the patient. This prevents the delivery of oxygenated blood to, and the removal of deoxygenated blood from, various physiological structures in the body of the patient. Without an effective oxygen supply for an extended period of time, cells in these physiological structures can become damaged or die. A rescuer, for instance, may perform chest compressions that circulate some blood through the body of the patient, which may deliver some oxygen to the physiological structures of the patient by forcing blood through the patient's blood vessels. A priority of chest compressions is to deliver oxygenated blood to the brain of the patient, because damage to the brain can have serious consequences to the patient. Accordingly, it is beneficial to monitor an amount of blood flowing inside, into, through, and out of the brain, particularly during the administration of chest compressions.

Unfortunately, it is difficult to monitor blood flow in the brain. In various cases, ultrasound-based techniques are used to monitor blood flow in other areas of the body. However, previous ultrasound-based technologies for detecting blood flow have several deficiencies for monitoring blood flow in the brain. In particular, ultrasound may be significantly attenuated and/or reflected by bone. Because the blood vessels in the brain are primarily disposed underneath the skull, the skull can prevent the ultrasound from being transmitted to, and reflected from, blood flowing through the brain. The skulls of adult patients, for instance, significantly block the transmission of ultrasound. Thus, previous technologies are unable to effectively image blood flow in the brain noninvasively using ultrasound.

Implementations of the present disclosure address these and other problems with previous technologies. In some examples, a noninvasive device transmits an incident beam (e.g., ultrasound and/or light) through one or more holes in the skull of a subject. The beam, for instance, is reflected from blood in one or more blood vessels in the brain and is detected by the device. By analyzing the return beam, the device may infer whether the blood is moving and/or amount of blood that is moving through the brain of the subject. These holes, for instance, include an orbital cavity, a nasal cavity, the temple, or the like. Thus, the incident beam can be used to effectively monitor blood flow in the brain of the subject, even in cases wherein the incident beam would otherwise be attenuated by the skull.

In some implementations, an amount of blood delivered to the brain is analyzed by monitoring blood in one or more carotid arteries and one or more jugular veins of a subject. For example, an incident beam is transmitted into the neck of the subject and is reflected by blood in the one or more carotid arteries and one or more jugular veins. By analyzing the reflection of the incident beam from the blood in the one or more carotid arteries, a device may identify blood flowing into the brain. By analyzing the reflection of the incident beam from the blood in the one or more jugular veins, the device may identify blood flowing out of the brain. In various cases, the device may infer an amount of blood flowing through the brain based on the amount of blood flowing into the brain and/or the amount of blood flowing out of the brain.

Various implementations of the present disclosure address problems in the technical field of cerebral monitoring and emergency care. Namely, some implementations described herein enable noninvasive techniques to be used to monitor and/or infer blood flow in the brain, despite the presence of the skull. Techniques described herein enable a rescuer to directly identify how much blood is being delivered to the brain of a patient, such as during chest compression administration. Accordingly, the rescuer can adjust parameters of the chest compressions to enhance cerebral blood flow, which can prevent the patient from experiencing brain damage or brain death during a cardiac emergency.

As used herein, the terms "blood flow," "blood flow parameters," and their equivalents, may refer to one or more physiological parameters indicative of a movement of blood through a blood vessel. For example, the term "blood velocity" may refer a change in position with respect to time (i.e., distance per time, such as meters per second) of one or more blood cells moving in a blood vessel. The term "blood speed" may refer to a magnitude of a velocity (i.e., distance per time) of one or more blood cells moving in a blood vessel. The term "velocity profile," for example, may refer to a velocity of blood in a blood vessel with respect to a location within the blood vessel, such as a velocity of blood in a blood vessel with respect to a location along a cross-section of the blood vessel. The term "flow rate," and its equivalents, may refer to a volume or mass of blood that passes a boundary (e.g., a cross-section of a blood vessel) with respect to time. The terms "net flow," "net flow volume," and their equivalents, may refer to a volume or mass of blood that passes a boundary during a discrete time interval, such as during a cardiac cycle. A net flow volume can be calculated by integrating a flow rate over the time interval. Unless otherwise specified explicitly or by context, the term "blood flow" may refer to blood velocity, blood speed, velocity profile, flow rate, net flow, or any other flow-related parameter described herein.

Implementations of the present disclosure will now be described with reference to the accompanying figures.

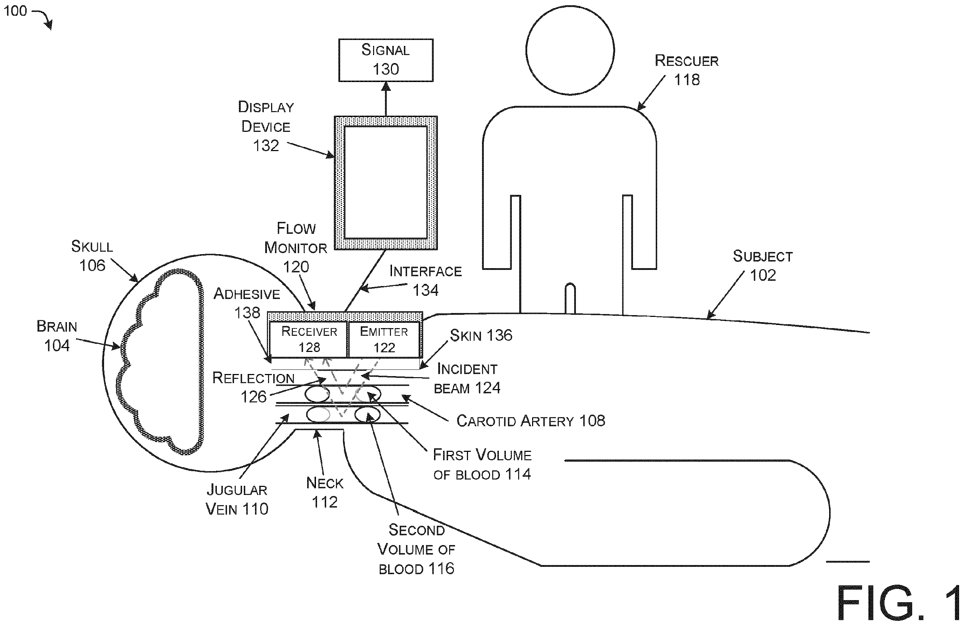

FIG. 1 illustrates an environment 100 for monitoring an amount of blood flowing through a brain 104 by directly monitoring blood flow in blood vessels traversing through the brain 104. In some implementations, the environment 100 includes a non-clinical location, such as the scene of a motor vehicle accident, a private residence, a public or private place such as a school, library, park, bus, bus station, airport, or airplane. For instance, the environment 100 is outside of a hospital, medical clinic, hospice, or other clinical setting.

A subject 102 is a person experiencing a medical emergency in the environment 100. As used herein, the term "medical emergency" and its equivalents, can refer to an acute illness, injury, symptom, or condition that puts life or long-term health at risk. Examples include cardiac arrest, respiratory arrest, stroke, ventricular fibrillation, seizure, drug overdose, drowning, electrocution, exposure to smoke, asphyxiation, or physical injury or insult. In particular cases, the subject 102 is experiencing cardiac arrest. The medical emergency may begin quickly and without warning such that the subject 102 is unable to reach a hospital or emergency room before experiencing severe health consequences. Accordingly, the subject 102 may receive rapid medical aid. As used herein, the term "medical aid" and its equivalents, can refer to Cardiopulmonary Resuscitation (CPR), activation of an emergency response system (e.g., calling for emergency services), chest compressions, defibrillation, and/or monitoring the physiological parameters of the subject 102.

A rescuer 118 is a person administering medical aid to the subject 102. The rescuer 118 may include a person within the environment 100, such as a bystander with or without medical training. In some cases, the rescuer 118 is a first responder, such as a firefighter or emergency medical technician, that enters the environment 100 responsive to the medical emergency.

The brain 104 of the subject 102 may be particularly vulnerable during the medical emergency. The brain 104 is a part of the central nervous system (CNS) of the subject 102. The brain 104 is an organ that controls various processes regulating the body of the subject 102, including movement, speech, perception, thought, memory, emotion, and the function of many other organs within the body. Tissue of the brain 104 includes neurons and other cells which utilize a supply of blood in order to function. Blood supplies oxygen and nutrients to cells of the brain 104.

However, in some examples, oxygenated blood may not be perfusing through the body of the subject 102 during the medical emergency. For example, if the subject 102 is in cardiac arrest, the heart of the subject 102 may not be effectively pumping blood through the body of the subject 102. Without a sufficient supply of oxygenated blood, cells in the brain 104 and other tissues can become damaged or die, which can lead to severe consequences for the subject 102 such as cognitive impairment, memory loss, loss of motor function and control, personality changes, speech difficulties, dysphagia, vision impairment, and brain death. Medical emergencies may cause impairment or inability of the subject 102 to breathe normally and/or transport blood to the brain 104. This in turn may result in hypoxemia, a low level of oxygen in the blood. In particular, the subject 102 may be at risk for cerebral hypoxia, in which tissues of the brain 104 do not receive enough oxygen to maintain homeostasis. Accordingly, determining whether the brain 104 of the subject 102 is receiving oxygenated blood may be highly pertinent to administering care to the subject 102.

Ultrasound- and light-based sensors can be used to monitor blood flow of the subject 102, such as blood flowing through blood vessels of the subject 102. For example, an ultrasound transducer can be used to detect the velocity of blood flowing through a blood vessel using a Doppler-based technique. As used herein, the term "ultrasound," and its equivalents, can refer to mechanical waves having a frequency in a range of 20 kilohertz (kHz) to 200 megahertz (MHz). Ultrasound, for example, is sound in a frequency that is greater than an upper detection limit of a human ear. Ultrasound can be transmitted through soft tissues, such as the skin of the subject 102, and toward blood flowing through blood vessel(s). When the transmitted ultrasound waves reach blood cells flowing through a blood vessel, the waves may be reflected. The reflection of an ultrasound wave has a frequency and/or phase shift with respect to the transmitted ultrasound wave. The frequency and/or phase shift is dependent on the velocity of the blood flowing through the blood vessel. A phase or frequency difference between the transmitted ultrasound wave and the reflection of the ultrasound wave may be referred to as a "Doppler shift." In various cases, the velocity of the blood may be derived based on the Doppler shift.

In some cases, a light source and photosensor can be used to detect the velocity through a blood vessel using Doppler-based techniques. In various cases, a light emitter is configured to output infrared light having a frequency in a range of 300 GHz - 430 THz and a wavelength in a range of 700 nanometers (nm) to 1 millimeter (mm). For example, the light emitter is configured to output near infrared light having a frequency in a range of 214 THz - 430 THz and a wavelength in a range of 750 nm to 1.4 micrometers (µm), short-wavelength infrared light having a frequency in a range of 100 THz - 214 THz and a wavelength in a range of 1.4-3 µm, mid-wavelength infrared light having a frequency in a range of 37 THz - 100 THz and a wavelength in a range of 3-8 µm, long-wavelength infrared light having a frequency in a range of 20 THz - 37 THz and a wavelength in a range of 8-15 µm, and/or far-infrared light having a frequency in a range of 0.3 THz - 20 THz and a wavelength in a range of 15-1000 µm. In some implementations, the light emitter is configured to output red light having a frequency in a range of 400 THz - 480 THz and a wavelength in a range of 625-750 nm. Light can be transmitted through soft tissues, such as the skin of the subject 102, and toward blood flowing through blood vessel(s). When the transmitted light waves reach blood cells flowing through a blood vessel, the light waves may be reflected. The reflection of a light wave has a frequency and/or wavelength shift with respect to the transmitted light. The frequency and/or wavelength shift is dependent on (e.g., proportional to) the velocity of the blood flowing through the blood vessel.

However, in various cases, it may be difficult to directly monitor blood flow in the brain 104 using ultrasound- and light-based sensors due to the presence of a skull 106 of the subject 102. The skull 106 is a cavernous bone structure in which the brain 104 is disposed. Bone, such as the skull 106, includes protein, collagen, and minerals such as calcium. The function of bone is to provide support and protection of soft tissues and organs such as the brain 104. Bone has a higher density and lower compressibility than soft tissues.

Ultrasound and light may be significantly attenuated and/or reflected by bone structures, such as the skull 106. For instance, mature bone (e.g., of an adult human) has high acoustic impedance due, at least in part, to its high density and low compressibility. Therefore, it can be difficult or impossible to transmit ultrasound and light through the skull 106, particularly if the subject 102 is an adult. Accordingly, the presence of the skull 106 makes it challenging to monitor blood flow in the brain 104 using ultrasound- and light-based sensors.

In various implementations of the present disclosure, a flow monitor 120 can be used to detect how much blood is going through the brain 104 without transmitting ultrasound and/or light through the skull 106. In particular cases, the flow monitor 120 infers a net flow volume of blood through the brain 104 of the subject 102 by monitoring blood flowing through a carotid artery 108 and/or blood flowing through a jugular vein 110 of the subject 102.

The flow monitor 120, for instance, is located outside of the body of the subject 102. That is, the flow monitor 120 may be an external, non-implantable device. The flow monitor 120 may be adhered to skin on the neck 112 of the subject 102 via an adhesive 138. In some implementations, the flow monitor 120 is disposed within a housing, for example an impact resistant shell, coating, or casing. The housing may be flexible or rigid, fixed or interchangeable, and may be impermeable to liquid. In some implementations, the flow monitor 120 is held on the skin by a strap, a buckle, a bandage, or some other fastener.

The carotid artery 108 and the jugular vein 110 are part of the circulatory system of the subject 102. The circulatory system includes a fluid circuit of various blood vessels (including the carotid artery 108 and the jugular vein 110). The heart of the subject 102, when functioning in non-emergency conditions, pumps blood through the blood vessels. In particular, the heart moves blood from lungs of the subject 102, where the blood can be oxygenated, to other portions of the body of the subject 102, such as the brain 104, other organs, and extremities of the subject 102. Along the circulatory system, cells within the blood deliver oxygen to cells of the subject 102, thereby supporting cellular respiration.

The carotid artery 108 is disposed in a neck 112 of the subject 102. In some cases, the carotid artery 108 is a blood vessel that supplies oxygenated blood to the brain 104 of the subject 102 from the lungs of the subject 102, such as before the subject 102 is in the medical emergency. Although FIG. 1 illustrates a single carotid artery 108, implementations are not so limited. For instance, the subject 102 may have multiple carotid arteries including the carotid artery 108. Arteries, such as the carotid artery 108, can have thick walls that include muscle tissue.

The jugular vein 110 is also disposed in the neck 112 of the subject 102. In some cases, the jugular vein 110 is a blood vessel that transports deoxygenated blood away from the brain 104, such as before the subject 102 is in the medical emergency. Although FIG. 1 illustrates a single jugular vein 110, implementations are not so limited. For instance, the subject 102 may have multiple jugular veins including the jugular vein 110. As used herein, the term "deoxygenated blood" and its equivalents, can refer to blood flowing through veins of the subject 102, blood from which oxygen bound to hemoglobin is released into the blood's plasma and absorbed into tissues and cells of the body, and blood with lower oxygen saturation or a lower ratio of the amount of oxygen bound to hemoglobin as compared to blood departing the lungs and flowing through arteries of the subject 102. Veins, such as the jugular vein 110, may have thinner walls compared to arteries and may include valves to maintain blood flow direction.

In particular cases, the flow monitor 120 includes one or more emitter(s) 122 configured to output one or more incident beams 124. As used herein, the term "beam," and its equivalents, may refer to a directional propagation of mechanical or electromagnetic energy from a source, such as mechanical waves or light. A beam, for instance, may propagate through media, such as air, water, soft tissue, or other materials. In implementations of the present disclosure, the incident beam 124 is propagated through soft tissue in the neck 112 of the subject 102. Accordingly, the skull 106 does not impede the incident beam 124. In various cases, the incident beam 124 is transmitted through the skin 136 of the subject 102.

In various cases, the incident beam 124 includes ultrasound waves. Emitter(s) 122 may include one or more piezoelectric crystals (including, e.g., lead zirconate titanate (PZT), LiNbO₃ (LN), lead magnesium niobate-lead titanate (PMN-PT), or lead indium niobate- lead magnesium niobate-lead titanate (PIN-PMN-PT)). When an electrical current is induced through the piezoelectric crystal(s) (e.g., by electrodes disposed on the piezoelectric crystal(s)), the piezoelectric crystal(s) vibrate at a frequency that produces ultrasound. In other instances, the ultrasound may be created by one or more micro-electro-mechanical systems (MEMS) devices. Any electrical to mechanical conversion system or material that operates at the ultrasound frequency range can be suitable. In various cases, the incident beam 124 includes infrared light having a frequency in a range of 300 GHz - 430 THz and a wavelength in a range of 700 nanometers (nm) to 1 millimeter (mm). For instance, the emitter(s) 122 includes one or more light sources, such as light-emitting diodes (LEDs) or lasers.

A portion of the incident beam 124 that does not propagate through and is not absorbed by media in the path of the incident beam 124 may be redirected, reflected, or otherwise scattered by the media and may become a reflection 126 of the incident beam 124. Media that may create a reflection 126 of the incident beam 124 include boundaries between media such as liquid, a soft tissue, bone, blood cells and plasma, and/or a contrast agent disposed in the blood stream. As used herein, the term "reflection," and its equivalents such as "refraction," "echo," or "scatter," may refer to a directional propagation of a portion of mechanical or electromagnetic energy from a beam that has been redirected by a medium. For example, the incident beam 124 is reflected by blood flowing through the carotid artery 108 and/or jugular vein 110, thereby producing the reflection 126. A discrepancy between the incident beam 124 and the reflection 126 with respect to frequency, wavelength, phase, or a combination thereof, is indicative of the velocity at which the blood is flowing.

Various contrast agents can be disposed in the blood to enhance the signal from the reflection 126. Ultrasound-based contrast agents, for instance, includes bubbles (e.g., bubbles including air, perfluorocarbons, or other gasses) that can be introduced into the bloodstream. The bubbles may have a diameter of 1 to 10 um. Surfactants, polymers, proteins, or other materials can be used as shells for the bubbles, in order to stabilize them in the bloodstream.

In some cases, the flow monitor 120 includes one or more receiver(s) 128 configured to detect the reflection 126 of the incident beam 124. According to various implementations, receiver(s) 128 may be configured to convert ultrasonic waves and/or light waves into analog electrical signals. Receiver(s) 128 may include one or more piezoelectric crystals (including, e.g., PZT, LN, PMN-PT, or PIN-PMN-PT). When mechanical stress, such as sound energy in the form of pressure waves, is applied to piezoelectric crystal(s), the piezoelectric crystal(s) vibrate and generate an electrical potential or voltage through the piezoelectric effect. In other instances, an electrical potential or voltage may be created by one or more micro-electro-mechanical systems (MEMS) devices. Any mechanical to electrical conversion system or material that operates at the ultrasound frequency range could be suitable. Receiver(s) 128 may include photodetectors and/or photosensors that detect light. Different types of light sensors may include photodiodes, photoresistors, phototransistors, and photovoltaic light sensors. Any system or material that that can be used to measure illuminance, respond to changes in the amount of light received, and/or convert light to electricity could be suitable.

According to some implementations, the flow monitor 120 includes one or more ultrasound transducers. As used herein, the terms "ultrasound transducer," "ultrasonic transducer," "transducer," and their equivalents, may refer to a device that generates or detects ultrasound. For instance, the ultrasound transducer(s) include the emitter(s) 122 and/or the receiver 128. In some cases, an ultrasound transducer includes a transducer element (e.g., a piezoelectric crystal, MEMS device, or another type of electrical-to-mechanical conversion device), a first electrode disposed on one side of the transducer element, and a second electrode disposed on another side of the transducer element. In various implementations, the ultrasound transducer is configured to produce ultrasound by inducing a current through or a voltage between the first and second electrodes. In some cases, the ultrasound transducer is configured to detect ultrasound by detecting a current through or voltage between the first and second electrodes that is induced when the ultrasound is received by the transducer element. In some cases, the ultrasound transducer is encased in a housing, which may be watertight. The ultrasound transducer, in some examples, further may include a matching layer that is disposed between the transducer element and a surface of the housing from which an incident beam of ultrasound is emitted. The matching layer may include a material having an acoustic impedance that is between the acoustic impedance of the transducer element and an acoustic lens (e.g., if one exists or between the transducer and skin). In some implementations, a gel layer is disposed between the housing of the ultrasound transducer and the skin 136 to prevent an impedance mismatch between the ultrasound transducer and the body (e.g., due to air between the flow monitor 120 and the skin 136), thereby preventing the incident beam 124 and/or the reflection 126 from being reflected by an interface 134 between the skin 136 and the ultrasound transducer. In some cases, the adhesive 138 serves as the gel layer.

The receiver(s) 128, in various implementations, are configured to generate an electrical signal indicative of the detected reflection 126. The flow monitor 120, in various cases, is configured to convert the electrical signal generated by the receiver(s) 128 into digital data that is indicative of the detected reflection 126. In some implementations, the flow monitor 120 may include one or more analog-to-digital converters (ADC). The flow monitor 120 may include one or more processors configured to analyze the digital data.

In various implementations, the flow monitor 120 is configured to determine a net volume of blood flow through the brain 104 of the subject 102 by analyzing the detected reflection 126. In some implementations, the flow monitor 120 determines a blood velocity across a cross-section of the carotid artery 108 and/or jugular vein 110 by identifying a Doppler shift (e.g., a frequency and/or phase shift) of the reflection 126 with respect to the incident beam 124. The Doppler shift may be dependent on, and proportional to, the velocity of blood flowing through the carotid artery 108 and/or jugular vein 110. In various implementations, the flow monitor 120 determines a blood volume flowing through the carotid artery 108 and/or jugular vein 110 identifying the velocity of blood flowing through a cross-section of the carotid artery 108 and/or jugular vein 110. By integrating the velocity of blood across the cross-sectional area of over a time interval, the flow monitor 120 may determine the volume of blood traversing the cross-section during the time interval.

In some implementations, the flow monitor 120 determines multiple blood volumes traversing multiple cross-sections of multiple blood vessels over the same time interval. For example, the flow monitor 120 may determine a first volume of blood 114 flowing towards the brain 104 through the carotid artery 108 during a time interval and a second volume of blood 116 flowing away from the brain 104 through the jugular vein 110 during the time interval. Thus, the flow monitor 120 may estimate an amount of blood flowing toward the brain 104 as well as an amount of blood flowing away from the brain 104 during the time interval. Either amount of blood, in various cases, may approximate an amount of blood flowing through the brain 104 during the time interval.

The time interval over which the first volume of blood 114 and the second volume of blood 116 are detected may be finite. In some cases, the time interval can have a length of 0.001 second, 0.01 second, 0.1 second, 1 second, 10 seconds, or the like. In some cases, the time interval is set based on a physiological process. For instance, the time interval may extend along a cyclical cycle of the subject 102. For instance, the time interval may have the length of a chest compression administered to the subject 102. In some cases, the time interval corresponds to a cardiac cycle and/or a VF cycle of the subject 102. A cardiac cycle is the activity of the heart from the beginning of one heartbeat to the beginning of the next heartbeat. In cases in which the subject 102 has a normal sinus rhythm (e.g., the subject 102 does not have an arrhythmia), the cardiac cycle includes a first diastole period where muscles of the heart relax and fill with blood from the body, and a second systole period, where muscles of the heart contract and pump blood out of the heart and to the body. The muscle activity of the heart can be monitored by detecting an electrocardiogram (ECG) of the subject 102, which may be graphically displayed as a waveform representing a relative electrical potential between at least one pair of electrodes disposed on either sides of the heart with respect to time. The cardiac cycle is a rhythmic sequence and may also be monitored and seen graphically in the form of a Wiggers diagram or venous pressure tracings. A VF cycle, for instance, may be defined as a time interval extending between adjacent peaks of an ECG that is indicative of VF. In some cases, a VF cycle is determined by identifying fluctuations of the ECG, which may occur 300 to 600 times per minute. In some cases, the VF cycle is determined by converting data indicative of the ECG to the frequency domain (e.g., by using a fast Fourier transform (FFT)), identifying a dominant frequency in the frequency domain representation of the data, and determining a period of the VF cycle by calculating the reciprocal of the dominant frequency.

In some cases, the time interval corresponds to a time at which a treatment is performed. For example, the time interval may be contemporaneous with a compression and/or release of a chest compression performed on the subject 102. The chest compression, for instance, is performed by a mechanical chest compression device or manually by the rescuer 118. In some cases, the time interval may correspond to a ventilation cycle of the subject 102. In some cases, the subject 102 may be breathing, such that the time interval corresponds to an inhale-exhale cycle of the subject 102. For instance, when the subject 102 is breathing, the ventilation cycle is the activity of the lungs from the beginning of one breath to the beginning of the next breath. The ventilation cycle includes a first inspiration period where intercostal muscles and muscles of the diaphragm contract, so the lungs fill with air, and a second expiration period, where intercostal muscles and muscles of the diaphragm relax and gasses flow out of the lungs. In some instances, the subject 102 is receiving assisted ventilation from an external device (e.g., a bag-valve mask (BVM) or ventilator), and the time interval corresponds to a time during which the external device administers a positive pressure ventilation cycle to the subject 102. In some cases, the external device reports the timing of the ventilation cycle to the flow monitor 120 in a communication signal.

In some implementations, the flow monitor 120 estimates blood flow through the brain 104 based on the first volume of blood 114 and the second volume of blood 116. The first volume of blood 114 and the second volume of blood 116 may provide independent estimates of the volume of blood flowing through the brain 104. The flow monitor 120 may be configured to determine a net flow volume of blood flowing through the brain 104, for example, by calculating an average of a measurement of the first volume of blood 114 and the second volume of blood 116, by repeating a measurement of the first volume of blood 114 and second volume of blood 116 several times and averaging the results, by taking a weighted average of the first volume of blood 114 and the second volume of blood 116 taking into account a relative of importance of each volume of blood, and/or by determining a quality of the first volume of blood 114 calculation and the second volume of blood 116 calculation based on a signal quality threshold parameter and selecting one flow volume over another.

The relative importance of a volume of blood measurement may be determined by evaluating random errors, noise and/or interference of each measurement. The signal quality threshold parameter may be, for example, a fixed value, a range of values representative of a standard deviation from a desired value, a value indicative of a physiological parameter of the subject 102, and/or a standard deviation of multiple flow volume measurements of the first volume of blood 114 and the second volume of blood 116. A measurement of the first volume of blood 114 and/or second volume of blood 116 is compared to the signal quality threshold parameter and if the measurement of the first volume of blood 114 and/or second volume of blood 116 is determined to be outside of the desired signal quality, the measurement may be omitted from calculations. Some medical aid administered to the subject 102 may interfere with the accuracy of measurements performed by the flow monitor 120, such as chest compressions or defibrillation. In various implementations, the flow monitor 120 may be configured to gate or exclude blood flow measurements that may contain interference or artifact from chest compression or defibrillation treatments. In some cases, the flow monitor 120 may be configured to selectively consider blood flow measurements detected during a treatment (e.g., chest compressions), in order to assess the effectiveness of the treatment.

In some cases, the flow monitor 120 outputs an indication of the net flow volume of blood through the brain 104. For instance, the flow monitor 120 includes a screen that visually presents a numeric indicator of the net flow of blood through (e.g., to and/or from) the brain 104.

According to various implementations, the flow monitor 120 can detect a return of spontaneous circulation (ROSC) of the subject 102. The flow monitor 120, for instance, can detect ROSC while the subject 102 is receiving chest compressions. When the subject 102 is receiving chest compressions, the chest compressions cause parallel or unidirectional blood flow in the carotid artery 108 and the jugular vein 110. In contrast, the pumping heart of the subject 102 circulating blood through the circulatory system causes antiparallel or bidirectional blood flow in the carotid artery 108 and the jugular vein 110. Thus, the flow monitor 120, in some cases, detects ROSC in response to detecting antiparallel or bidirectional blood flow in the carotid artery 108 and jugular vein 110.

In some cases, the flow monitor 120 detects ROSC using other techniques. In general, the heart is capable of pumping blood at a greater peak velocity than chest compressions. Therefore, the monitor 120 may detect ROSC by detecting that a blood flow parameter (e.g., blood velocity or net blood flow volume) through the carotid artery 108 or the jugular vein 110 is greater than a threshold. Further, the blood flow parameter through the carotid artery 108 or the jugular vein 110 with respect to time during chest compressions is different than the blood flow parameter through the carotid artery 108 or the jugular vein 110 with respect to time during ROSC. According to some implementations, the flow monitor 120 detects ROSC by determining that at least one frequency component of the blood flow parameter over time through the carotid artery 108 or jugular vein 110 has changed. For instance, a shape or morphology of the flow rate over time can be indicative of ROSC. Blood flow-based ROSC detection may be particularly useful in scenarios in which the subject 102 may have pulseless electrical activity (PEA) or pseudo-PEA. During PEA, the ECG of the subject 102 may be consistent with circulation (e.g., the ECG may have characteristics of a normal sinus rhythm), however, the heart may nevertheless be not pumping blood effectively throughout the body of the subject 102. Thus, it may be difficult (or even impossible) to detect spontaneous circulation by analyzing the ECG of the subject 102, alone. By detecting the blood flow parameter, the flow monitor 120 may detect ROSC, or the absence of ROSC, accurately, regardless of the characteristics of the ECG of the subject 102.

In some cases, the flow monitor 120 provides feedback about the efficacy of the chest compressions based on the flow velocity through the carotid artery 108 and jugular vein 110 and/or the net flow volume of blood through the brain 104 of the subject 102. For instance, if the flow monitor 120 detects less than a threshold blood velocity through the carotid artery 108 and jugular vein 110, or detects less than a threshold net flow volume through the brain 104 of the subject 102 (e.g., during a particular time period), the flow monitor 120 can output a signal 130 via a display device 132. The signal 130 may indicate the estimated net flow volume of blood through the brain 104 of the subject 102. In some cases, the signal 130 is an alert output to the rescuer 118 performing the chest compressions and may cause the rescuer 118 to adjust the depth of the chest compressions, adjust the frequency of the chest compressions, or adjust the position of the chest compressions relative to the chest of the subject 102.

In some implementations, the signal 130 is transmitted to a mechanical chest compression device that is performing the chest compressions and/or an external defibrillator (not shown).

By detecting blood flow in the brain 104, the flow monitor 120 is configured to detect an efficacy of chest compressions being administered to the subject 102. Technologies that monitor blood flow in extremities can indirectly detect chest compression efficacy. However, because a primary purpose of chest compressions is to provide oxygenated blood to the brain 104 of the subject 102, the flow monitor 120 is able to directly detect the efficacy of the chest compressions by monitoring blood flow in the brain 104. The flow monitor 120, for instance, can output a feedback signal (e.g., to the mechanical chest compression device or to the rescuer 118) indicating whether chest compressions are generating blood flow in the brain 104. In some cases, the feedback signal includes a metric indicative of the blood flow to the brain 104 (e.g., a net flow volume of blood through the carotid artery 108 and/or jugular vein 110).

An example rescue situation will now be described with respect to FIG. 1. Within a public environment 100, a subject 102 begins experiencing VF and collapses. A rescuer 118, who is an emergency medical technician, enters the environment 100 and comes to the aid of the subject 102. The rescuer 118 determines that the subject 102 should receive immediate medical aid in the environment 100. The rescuer 118 applies the flow monitor 120 to the neck 112 of the subject 102, and immediately begins administering manual chest compressions to the subject 102. The flow monitor 120 detects the efficacy of the chest compressions by determining a net flow volume of blood through (e.g., to and/or from) the brain 104 of the subject 102 over one or more time periods (e.g., during a time period including one or more chest compressions). The flow monitor 120 determines that the net flow volume of blood to the brain 104 is insufficient and the flow monitor 120 instructs the rescuer 118 via the display device 132 to increase the depth of the chest compressions. In response, the rescuer 118 may increase the depth of the chest compressions and improve circulation to the brain 104 of the subject 102. After a CPR period expires (e.g., after 2 minutes of administering chest compressions), the rescuer 118 uses a defibrillator to administer an electrical shock to the subject 102. The flow monitor 120 detects ROSC after the electrical shock is administered, and outputs an indication of the ROSC to the rescuer 118. Accordingly, the rescuer 118 may refrain from administering additional chest compressions.

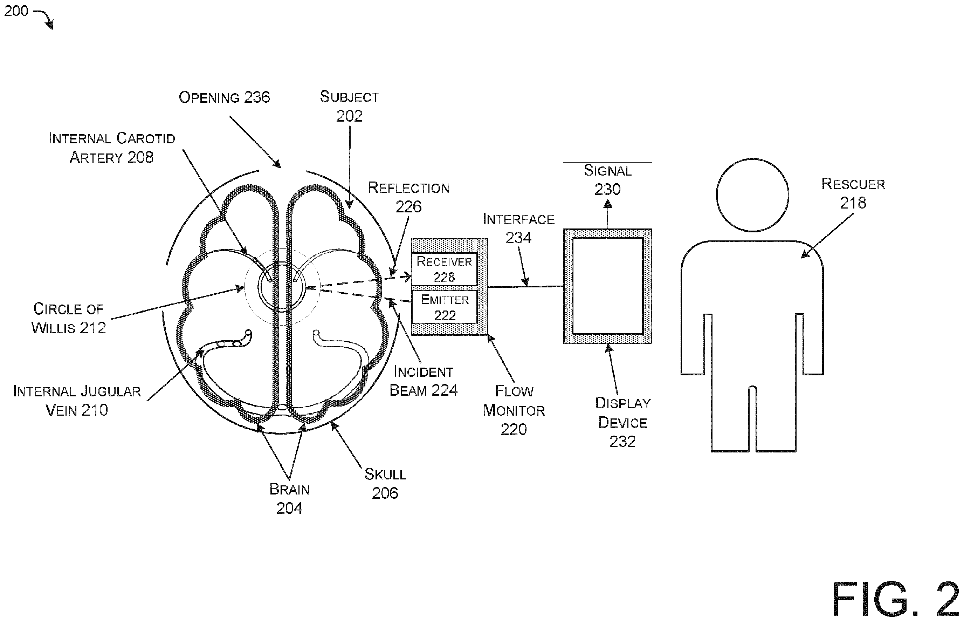

FIG. 2 illustrates an environment 200 for monitoring blood flow in a brain 204 of a subject 202 by monitoring blood vessels in the brain 204 through an opening 236 in a skull 206 of the subject. In some implementations, the environment 200 includes a non-clinical location, such as the scene of a motor vehicle accident, a private residence, a public or private place such as a school, library, park, bus, bus station, airport, or airplane. For instance, the environment 200 is outside of a hospital, medical clinic, hospice, or other clinical setting.

A subject 202 is a person experiencing a medical emergency in the environment 200. The blood through the brain 204 of the subject 202 may be monitored.

A rescuer 218 is a person administering medical aid to the subject 202. The rescuer 218 may include a person within the environment 200, such as a bystander with or without medical training. In some cases, the rescuer 218 is a first responder, such as a firefighter or emergency medical technician, that enters the environment 200 responsive to the medical emergency.

However, in some examples, oxygenated blood may not be perfusing through the body of the subject 202 during the medical emergency. For example, if the subject 202 is in cardiac arrest, the heart of the subject 202 may not be effectively pumping blood through the body of the subject 202. The brain 204 of the subject 202 may be particularly vulnerable during the medical emergency. Without a sufficient supply of oxygenated blood, cells in the brain 204 and other tissues can become damaged or die, which can lead to severe consequences for the subject 202, such as cognitive impairment, memory loss, loss of motor function and control, personality changes, speech difficulties, dysphagia, vision impairment, and brain death.

Ultrasound- and light-based sensors can be used to monitor blood flow of the subject 202, such as blood flowing through blood vessels of the subject 202. However, in various cases, it may be difficult to directly monitor blood flow in the brain 204 using ultrasound- and light-based sensors due to the presence of the skull 206 of the subject 202. For instance, bone has high acoustic impedance due, at least in part, to the high density and low compressibility of the bone. Therefore, it can be difficult to transmit ultrasound and light through the skull 206. Accordingly, the presence of the skull 206 makes it challenging to monitor blood flow in the brain 204 using ultrasound- and light-based sensors.

In various implementations of the present disclosure, a flow monitor 220 can be used to detect blood flow through the brain 204 without transmitting ultrasound and/or light through the skull 206. The flow monitor 220 may transmit ultrasound and/or light through an opening 236 in the skull 206 of the subject 202. The opening 236 is a natural orifice, window, gap, or hole of the skull 206 that provides energetic access to blood vessels of the brain 204. Energetic access includes the ability for ultrasound and/or light wave energy to pass through soft tissue that surrounds and passes into the skull through the opening 236.

The flow monitor 220, for instance, is located outside of the body of the subject 202. That is, the flow monitor 220 may be an external, non-implantable device. The flow monitor 220 may be adhered to skin of the subject 202 via an adhesive 238. In some implementations, the flow monitor 220 is disposed within a housing, for example an impact resistant shell, coating, or casing. The housing may be flexible or rigid, fixed or interchangeable, and may be impermeable to liquid. In some implementations, the flow monitor 220 is held on the skin by a strap, a buckle, a bandage, or some other fastener.

There may be multiple openings 236 in the skull 206, such as an orbit, ear, nose and/or temple. An orbit may provide energetic access to one or more blood vessels of the brain, such as an ophthalmic artery, a central retinal artery, an internal carotid artery 208, ophthalmic veins (vortex veins), and a central retinal vein, through the inferior orbital fissure, the superior orbital fissure, and/or the optic foramen. The ear may provide energetic access to one or more blood vessels of the brain, including the internal carotid artery 208 and an internal jugular vein 210 through the auditory canal. The nose may provide energetic access to one or more blood vessels of the brain, including the internal carotid artery 208 and the internal jugular vein 210 through the or nasal cavity.

In particular cases, the flow monitor 220 infers a net flow of blood through the brain 204 of the subject 202 by monitoring blood flowing through blood vessels of the brain, including an internal carotid artery 208 and an internal jugular vein 210 of the subject 202. The presence or absence of a net flow of blood in the brain 204 can be relevant for monitoring, for example, an efficacy of chest compressions and a ROSC of the subject 202. In particular cases, the flow monitor 220 infers a net flow of blood through the brain 204 of the subject 202 by monitoring blood flowing through any blood vessel, or any combination of blood vessels, of the brain 204 of the subject 202.

A Circle of Willis 212 is a region on the inferior side of the brain 204 where blood vessels join together to supply oxygen to the brain. The Circle of Willis 212 is disposed within the skull 206. An opening 236 may provide energetic access to blood vessels within the Circle of Willis 212. For example, the internal carotid artery 208, an anterior communicating artery, a middle cerebral artery, a posterior communicating artery, and a posterior cerebral artery may be disposed within the Circle of Willis 212 of the subject 202. In some cases, the internal carotid artery 208 is a blood vessel that supplies oxygenated blood to the brain 204 of the subject 202 from the lungs of the subject 202, such as before the subject 202 is in the medical emergency. Although FIG. 2 illustrates the internal carotid artery 208, implementations are not limited to the use of the internal carotid artery 208. For instance, the subject 202 may have multiple carotid arteries including an external carotid artery (not illustrated) and the internal carotid artery 208.

An internal jugular vein 210 is disposed within the skull 206 the subject 202. In some cases, the internal jugular vein 210 is a blood vessel that transports deoxygenated blood away from the brain 204, such as before the subject 202 is in the medical emergency. Although FIG. 2 illustrates a single internal jugular vein 210, implementations are not limited to the use of the internal jugular vein. For instance, the subject 202 may have multiple jugular veins including an external jugular vein and the internal jugular vein 210.

In particular cases, the flow monitor 220 includes one or more emitter(s) 222 configured to output one or more incident beam(s) 224. In implementations of the present disclosure, the incident beam 224 is propagated through the opening 236 of the subject 202. In various examples, the opening 236 of the subject 202 is a natural orifice of the skull 206 that provides energetic access to blood vessels of the brain 204. Accordingly, the skull 206 does not impede the incident beam 224 if it is transmitted through the opening 236. In various cases, the incident beam 224 is transmitted through the skin of the subject 202. According to some examples, light may be transmitted through the skull 206. For example, if the incident beam 224 includes light (e.g., VNIR light), the incident beam 224 may be transmitted to one or more blood vessels in the brain 204 without necessarily being transmitted through the opening 236.

In various cases, the incident beam 224 includes ultrasound waves. Emitter(s) 222 may include one or more piezoelectric crystals. In various cases, the incident beam 224 includes infrared light. For instance, the emitter(s) 222 includes one or more light sources, such as LEDs or lasers.

A portion of the incident beam 224 that does not propagate through and is not absorbed by media in the path of the incident beam 224 may be redirected, reflected, or scattered by the media and may become a reflection 226 of the incident beam 224. A discrepancy between the incident beam 224 and the reflection 226 with respect to frequency, wavelength, phase, or a combination thereof, is a Doppler shift indicative of the velocity at which the blood is flowing. The Doppler shift, for instance, is detected by the flow monitor 220 based on the reflection 226.

The flow monitor 220 includes one or more receiver(s) 228 configured to detect one or more reflection(s) 226 of the incident beam(s) 224. According to various implementations, receiver(s) 228 may be configured to convert ultrasonic waves and/or light waves into analog electrical signals. Receiver(s) 228 may include one or more piezoelectric crystals (including, e.g., PZT, LN, PMN-PT, or PIN-PMN-PT). Receiver(s) 228 may include photodetectors and photosensors that detect light. Different types of light sensors may include photodiodes, photoresistors, phototransistors, and photovoltaic light sensors. In some cases, interferometric sensors are used. Any system or material that that can be used to measure illuminance, respond to changes in the amount of light received, and/or convert light to electricity could be suitable.

According to various implementations, the flow monitor 220 includes one or more ultrasound transducers. In some implementations, a gel layer is disposed between the housing of the ultrasound transducer and the skin that further matches the impedance between the ultrasound transducer and the body, thereby preventing the incident beam(s) 224 and/or the reflection(s) 226 from being reflected by air present at an interface 234 between the skin and the ultrasound transducer. In some cases, the adhesive 238 serves as the gel layer.

The receiver(s) 228, in various implementations, are configured to generate an electrical signal indicative of the detected reflection 226. The flow monitor 220, in various cases, is configured to convert the electrical signal generated by the receiver(s) 228 into digital data that is indicative of the detected reflection 226. In some implementations, the flow monitor 220 may include one or more ADC. The flow monitor 220 may include one or more processors configured to analyze the digital data.

In some cases, the flow monitor 220 may be unable to distinguish what structures have reflected the detected reflection 226. For instance, a complex tangle of blood vessels may be present within the brain 204, such as within the Circle of Willis 212. In various cases, the detected reflection 226 may be output by multiple blood vessels, configured to carry blood in various directions within the brain 204, and which may be disposed at different angles. Nevertheless, in some examples, the flow monitor 220 may be configured to detect the presence of blood flow in the brain 204 based on the detected reflection 226, even if the detected reflection 226 is emitted from multiple blood vessels.

According to various implementations, the flow monitor 220 is configured to detect the presence of blood movement in the brain 204 based on the Doppler shift of the detected reflection 226 with respect to the incident beam 224. It may be inferred that any movement causing the Doppler shift within the brain 204 is caused by blood movement. Thus, the blood monitor 220 may qualitatively determine that blood is moving within the brain 204 by determining that the detected reflection 226 has a Doppler shift with respect to the incident beam 224.

In some cases, it is possible for the flow monitor 220 to quantify the amount of blood flow in the brain 204 using similar techniques to those described above with respect to FIG. 1. For example, in some examples, blood in one or more individual blood vessels (e.g., the internal carotid artery 208 and/or the internal jugular vein 210) reflects the detected reflection 226. Based on the Doppler shift between the incident beam 224 and the detected reflection 226, the flow monitor 220 may detect a velocity of blood in an individual blood vessel, a velocity profile along a cross-section of the individual blood vessel, a volumetric flow rate through the blood vessel, a net flow volume through the individual blood vessel, or some other flow parameter through the individual blood vessel. The flow monitor 220, for instance, quantifies an amount of blood flow in the brain 204 by measuring the flow parameter through one or more individual blood vessels in the brain 204.

In various implementations, the flow monitor 220 is configured to qualitatively or quantitatively detect blood flow in the brain 204 during one or more time intervals. In some cases, the time interval can have a length of 0.001 second, 0.01 second, 0.1 second, 1 second, 10 seconds, or the like. In some cases, the time interval is set based on a physiological process. For instance, the time interval may extend along a cardiac cycle or natural ventilation cycle of the subject 202. In some cases, the time interval corresponds to a time at which a treatment (e.g., a chest compression, assisted ventilation, pacing, or the like) is performed.

Qualitative measurements of blood flow in the brain 204, in some cases, enable the flow monitor 220 to detect spontaneous circulation (e.g., ROSC) and/or chest compression efficacy. For instance, if the flow monitor 220 detects blood flow during a time interval in which chest compressions are not being administered to the subject 202, then the flow monitor 220 may infer that blood is spontaneously circulating in the body of the subject 202. In some examples, the flow monitor 220 detects spontaneous circulation by detecting blood flow that is temporally aligned (although possibly with a time delay) with one or more QRS complexes detected in an ECG of the subject 202. In some cases, the flow monitor 220 detects chest compression efficacy by detecting the presence of blood flow during a time interval in which one or more chest compressions are being administered. Relatedly, the flow monitor 220 may determine that chest compressions are not effective by detecting the absence of detectable blood flow in the brain 204 during a time interval in which one or more chest compressions are being administered. In some cases, the flow monitor 220 determines when chest compressions are being administered based on a communication signal received from an external device (e.g., a monitor-defibrillator, a chest compression sensor, a mechanical chest compression device that is administering chest compressions, or any combination thereof.)

The flow monitor 220, in some examples, is configured to generate a pseudo-velocity profile based on the Doppler shift between the detected reflection 226 and the incident beam 224. For instance, the flow monitor 220 may generate a velocity profile as though the detected reflection 226 is reflected from blood through a single blood vessel. Because the detected reflection 226 may emanate from multiple blood vessels, carrying blood in different directions, the pseudo-velocity profile may not accurately represent blood flow through any single blood vessel in the brain 204. In some cases, the flow monitor 220 generates a pseudo net flow volume by integrating the pseudo velocity profile with respect to area (e.g., an arbitrary area within the brain 204, such as a cross-section including at least apportion of the Circle of Willis 212), and with respect to time (e.g., over a time interval). In particular cases, the pseudo net flow volume is detected over a cardiac cycle, chest compression, or other time interval. In some implementations, the flow monitor 220 detects a pseudo net flow volume during a first phase of a cyclical event (e.g., a compression phase of a chest compression) and a pseudo net flow volume during a second phase of the cyclical event (e.g., a decompression phase of the chest compression), and may compare the pseudo net flows to detect whether the chest compression is effective. For example, if the pseudo net flow volume during the compression phase of the chest compression has an equivalent magnitude but is in the opposite direction of the pseudo net flow volume during the decompression phase of the chest compression, then the flow monitor 220 may determine that the chest compression is producing sloshing in the brain 204 without net flow. This may indicate that the chest compression is ineffective. If, however, the flow monitor 220 detects greater pseudo flow (e.g., a greater pseudo net flow) magnitude during the compression phase than the decompression phase, then the flow monitor 220 may infer that the chest compression is effectively producing a net flow volume of blood through the brain 204.

In some cases, the flow monitor 220 outputs an indication of detected blood flow in the brain 204. For instance, the flow monitor 220 includes a screen that visually presents a numeric indicator of the net flow of blood to the brain 204. In some cases, the flow monitor 220 presents a qualitative indicator of the net flow of blood to the brain 204. For instance, the qualitative indicator may be a first symbol, color, brightness, or size if the net flow of blood to the brain 204 is present and/or above a threshold, and may be a second symbol, color, brightness, or size if the net flow of blood to the brain 204 is absent and/or below the threshold.

According to various implementations, the flow monitor 220 can detect spontaneous circulation in the brain 204 of the subject 202. The flow monitor 220, for instance, can detect spontaneous circulation while the subject 202 is receiving chest compressions. When the subject 202 is receiving chest compressions, the chest compressions may cause primarily parallel or unidirectional blood flow in the internal carotid artery 208 and the internal jugular vein 210. In contrast, the pumping heart of the subject 202 circulating blood through the circulatory system may cause primarily antiparallel or bidirectional blood flow in the internal carotid artery 208 and the internal jugular vein 210. Thus, the flow monitor 220, in some cases, detects spontaneous circulation in response to detecting antiparallel or bidirectional blood flow in the internal carotid artery 208 and internal jugular vein 210.

In some cases, the flow monitor 220 detects spontaneous circulation using other techniques. In general, the heart is capable of pumping blood at a greater peak velocity than chest compressions. Therefore, the flow monitor 220 may detect spontaneous circulation by detecting that a blood velocity, pseudo blood velocity, volumetric flow rate, pseudo volumetric flow rate, or other blood flow parameter through at least one blood vessel of the brain 204, such as the internal carotid artery 208, the internal jugular vein 210, or one or more blood vessels within the Circle of Willis 212, is greater than a threshold. Further, a waveform representing the blood flow parameter with respect to time may be different during chest compressions than during spontaneous circulation. According to some implementations, the flow monitor 220 detects spontaneous circulation by determining that at least one frequency component of the flow parameter over time has changed. For instance, a shape or morphology of the flow rate over time can be indicative of spontaneous circulation.

In some cases, the flow monitor 220 provides feedback about the efficacy of the chest compressions based on detecting blood flow in the brain 204 of the subject 202. For instance, if the flow monitor 220 detects less than a threshold blood flow parameter, , the flow monitor 220 can output a signal 230 via a display device 232. The signal 230 may indicate the flow parameter, qualitatively and/or quantitatively. In particular cases, the signal 230 may be indicative of an aggregate blood flow of multiple blood vessels. In some cases, the signal 230 is output to the rescuer 218 performing the chest compressions and may cause the rescuer 218 to adjust a chest compression parameter, such as to adjust the depth of the chest compressions, adjust the frequency of the chest compressions, or adjust the position of the chest compressions relative to the chest of the subject 202.

In some implementations, the signal 230 is transmitted to a mechanical chest compression device that is performing the chest compressions and/or a defibrillator (not shown). For instance, the mechanical chest compression device may be configured to change one or more chest compression parameters in response to the signal 230.

By detecting blood flow in the brain 204, the flow monitor 220 is configured to detect an efficacy of chest compressions being administered to the subject 202. Technologies that monitor blood flow in extremities can indirectly detect chest compression efficacy. However, because a primary purpose of chest compressions is to provide oxygenated blood to the brain 204 of the subject 202, the flow monitor 220 is able to directly detect the efficacy of the chest compressions by monitoring blood flow in the brain 204. The flow monitor 220, for instance, can output a feedback signal (e.g., to the mechanical chest compression device or to the rescuer 218) indicating whether chest compressions are generating blood flow in the brain 204. In some cases, the feedback signal includes a metric indicative of the blood flow to the brain 204 (e.g., a net flow volume through the internal carotid artery 208 and/or internal jugular vein 210).

An example rescue situation will now be described with respect to FIG. 2. Within a public environment 200, a subject 202 begins experiencing VF and collapses. A rescuer 218, who is an emergency medical technician, enters the environment 200 and comes to the aid of the subject 202. The rescuer 218 determines that the subject 202 should receive immediate medical aid in the environment 200. The rescuer 218 applies the flow monitor 220 to a temple of the subject 202, and immediately begins administering manual chest compressions to the subject 202. For instance, the flow monitor 220 may be applied as a headband. The flow monitor 220 detects the efficacy of the chest compressions by detecting whether blood is flowing through the brain 204 of the subject 202 during the chest compressions. The flow monitor 220 determines that the net flow of blood to the brain 204 is insufficient and the flow monitor 220 instructs the rescuer 218 via the display device 232 to increase the depth of the chest compressions. In response, the rescuer 218 may increase the depth of the chest compressions and improve circulation in the brain 204 of the subject 202. After a CPR period expires (e.g., after 2 minutes of administering chest compressions), the rescuer 218 uses a defibrillator to administer an electrical shock to the subject 202. The flow monitor 220 detects ROSC after the electrical shock is administered, and outputs an indication of the ROSC to the rescuer 218. Accordingly, the rescuer 218 may refrain from administering additional chest compressions.

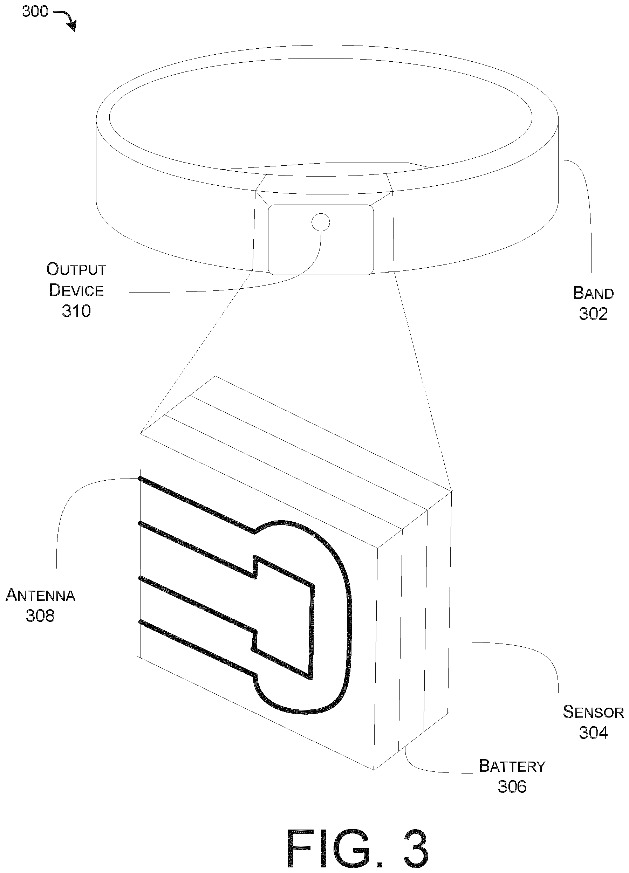

FIG. 3 illustrates the structure of an example flow monitor 300. In some cases, the flow monitor 300 is a patch that can be strapped to a subject or adhered to the skin of the subject. In some implementations, the flow monitor 300 includes the flow monitor 120 and/or the flow monitor 220. In various cases, the flow monitor 300 includes a band 302 configured to be disposed around an appendage of the subject. According to some examples, the band 302 is configured to be disposed around a chest, abdomen, head, or neck of the subject.

In various implementations, the flow monitor 300 includes a sensor 304 configured to detect a physiological parameter indicative of blood flow through a subject. In some cases, the sensor 304 includes a microphone configured to detect a sound generated by the body of the subject. In some examples, the sensor 304 includes an ultrasound transducer configured to detect the velocity of blood flowing through at least one blood vessel of the subject and/or through the heart of the subject. In some examples, the sensor 304 includes an ultrasound transducer configured to detect heart wall motion. In some cases, the sensor 304 includes at least one transmitter and/or at least one receiver. In some examples, the sensor 304 includes one or more accelerometers.

The sensor 304, for instance, is powered by a battery 306. In some cases, the battery 306 is disposable. In some examples, the battery 306 is rechargeable.

The flow monitor 300, in various cases, further includes an antenna 308. The antenna 308, in various implementations, is configured to transmit and/or receive communication signals from an external device. In some cases, the antenna 308 enables the flow monitor 300 to communicate with another flow monitor or another type of medical device (e.g., an AED or monitor-defibrillator). For instance, the flow monitor 300 is configured to transmit, to an external device, an indication of the physiological parameter detected by the sensor 304. The antenna 308, in various cases, is powered by the battery 306.

According to various examples, the sensor 304, the battery 306, and the antenna 308 are arranged in respective layers of the flow monitor 300. For example, each layer may correspond to a circuit board (e.g., a PCB) that accommodates a respective component. In various cases, the layer corresponding to the sensor 304 is configured to be adjacent to the subject when the flow monitor 300 is disposed on the subject. The layer corresponding to the antenna 308, for example, faces away from the subject when the flow monitor 300 is disposed on the subject. Accordingly, the body of the subject may be prevented from interfering with wireless signals transmitted and/or received by the antenna 308.

In some examples, the flow monitor 300 further includes an output device 310. The output device 310 is configured to output a signal to a user. In some cases, the signal is based on the physiological parameter detected by the sensor 304. For instance, the output device 310 includes a light that is activated when a blood velocity detected by the sensor 304 remains below a threshold blood velocity for greater than a threshold period of time.

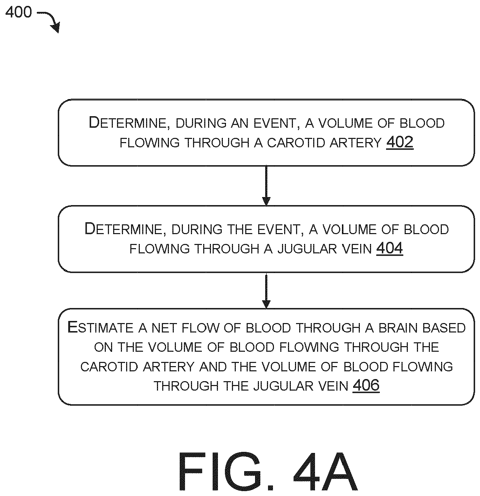

FIG. 4A illustrates an example process 400 for determining a net flow of blood though a brain of a subject. In various examples, the process 400 can be performed by an entity. The entity may include the flow monitor 120, the display device 132, the flow monitor 220, the display device 232, a medical device, a resuscitation system, an external flow monitor, a monitor-defibrillator, a mechanical chest compression device, a computing device, at least one processor, or any combination thereof. In some cases, the entity may include a housing and an adhesive may be disposed on the housing and configured to attach the entity to the body of the subject.

At 402 the entity determines, during an event, a volume of blood flowing through a carotid artery of a subject toward a brain of the subject. The event may include a chest compression, a cardiac cycle, or a ventilation cycle. In some cases, the volume of blood flowing through a carotid artery is flowing away from the heart of the subject. The entity may output a first incident beam from a first emitter. The first incident beam may include ultrasound or light. In some cases, the ultrasound may have a frequency in a range of 20 kilohertz (kHz) to 200 megahertz (MHz). For example, the ultrasound may have a frequency in a range of 100 kHz to 2 MHz. In some cases, the incident beam includes infrared light having, for example, a frequency in a range of 300 GHz - 430 THz and a wavelength in a range of 700 nanometers (nm) to 1 millimeter (mm). The entity may include a first receiver. In some cases, the first receiver is configured to generate a first analog signal by detecting a reflection of the first incident beam from the blood flowing through the carotid artery. The first incident beam may be reflected from blood, including blood cells and plasma, within the carotid artery, and/or a contrast agent disposed in the blood flowing through the carotid artery. The entity may include a first analog to digital converter (ADC) configured to convert the first analog signal into a first digital signal. Alternatively, the first receiver may be configured to receive a data signal representative of a reflection of the first incident beam from the blood flowing through the carotid artery. The received data signal may be the first digital signal, or may be converted into the first digital signal. The entity may include one or more processors configured to analyze the digital data. In some cases, the entity determines a volume of blood flowing through the carotid artery by analyzing the first digital signal. The entity may, for example, determine a volume of blood flowing through the carotid artery by identifying a velocity of the blood flowing through the carotid artery and integrating the velocity of the blood flowing through the carotid artery with respect to time. In some cases, the entity may identify a Doppler shift of the reflection of the first incident beam with respect to the first incident beam. The entity may determine the velocity of the blood flowing through the carotid artery by analyzing the Doppler shift of the reflection of the first incident beam with respect to the first incident beam.

At 404 the entity determines, during the event, a volume of blood flowing through a jugular vein of the subject away from the brain of the subject. In some cases, the volume of blood flowing through the jugular vein is flowing towards the brain of the subject. For example, blood may flow through the jugular vein and toward the brain of the subject when the subject is receiving a chest compression. The entity may output a second incident beam from a second emitter. The second incident beam may include ultrasound or light. In some cases, the ultrasound may have a frequency in a range of 20 kilohertz (kHz) to 200 megahertz (MHz). For example, the ultrasound may have a frequency in a range of 100 kHz to 2 MHz. In some cases, the incident beam includes infrared light having, for example, a frequency in a range of 300 GHz - 430 THz and a wavelength in a range of 700 nanometers (nm) to 1 millimeter (mm). The entity may include a second receiver. In some cases, the second receiver is configured to generate a second analog signal by detecting a reflection of the second incident beam from the blood flowing through the jugular vein. The second incident beam may be reflected from blood, including blood cells and plasma, within the jugular vein, and/or a contrast agent disposed in the blood flowing through the jugular vein. The entity may include a second ADC configured to convert the second analog signal into a second digital signal. In some cases, the entity determines a volume of blood flowing through the jugular vein by analyzing the second digital signal. The entity may, for example, determine a volume of blood flowing through the jugular vein by identifying a velocity of the blood flowing through the jugular vein and integrating the velocity of the blood flowing through the jugular vein with respect to time. In some cases, the entity may identify a Doppler shift of the reflection of the second incident beam with respect to the second incident beam. The entity may determine the velocity of the blood flowing through the jugular vein by analyzing the Doppler shift of the reflection of the second incident beam with respect to the second incident beam. In particular embodiments, the entity may determine that the blood flows through the jugular vein toward the brain of the subject when the subject is receiving the chest compression.

At 406 the entity, for example, may estimate a net flow of blood through the brain of the subject based on the volume of blood flowing through the carotid artery and the volume of blood flowing through the jugular vein. In some cases, the net flow of blood through the brain may be estimated based on the volume of blood flowing from the brain of the subject and the volume of blood flowing toward the brain of the subject. The net flow of blood through the brain may be estimated, for example, by averaging the volume of blood flowing through the carotid artery and the volume of blood flowing through the jugular vein. Averaging may include adding the volume of blood flowing through the carotid artery and the volume of blood flowing through the jugular vein and dividing the sum by the total number of measurements. In some cases, one or more measurements of the volume of blood flowing through the carotid artery and the volume of blood flowing through the jugular vein may be used to determine an average. In some cases, averaging may include taking a weighted average of the volume of blood flowing through the carotid artery and the volume of blood flowing through the jugular vein.

The entity may perform various additional functions based on the net flow of blood through the brain. The entity may output a signal indicative of the net flow of blood through the brain of the subject. The entity may, for example, include a display to visually output the signal. The signal may be output to a user, to a computing device, such as a mobile device, and/or to a medical device such as, a defibrillator, or a mechanical chest compression device. The entity may include an output device configured to transmit the signal indicative of the net flow of blood through the brain of the subject to a computing device, such as a mobile device, and/or to a medical device, such as a defibrillator or a mechanical chest compression device. In some cases, the output device may be a transceiver. In some cases, the entity may determine that the net flow of blood through the brain is below a threshold. The entity may determine an efficacy of chest compressions by comparing the net flow of blood through the brain of the subject to the threshold. For example, the entity determines the efficacy of the chest compression by determining that the net flow of blood through the brain is below the threshold. In some cases, the signal may include the efficacy of chest compressions. In response to determining that the net flow of blood through the brain is below the threshold, the entity may, for example, output instructions to initiate chest compressions, or change a position, frequency, timing, or depth of chest compressions.

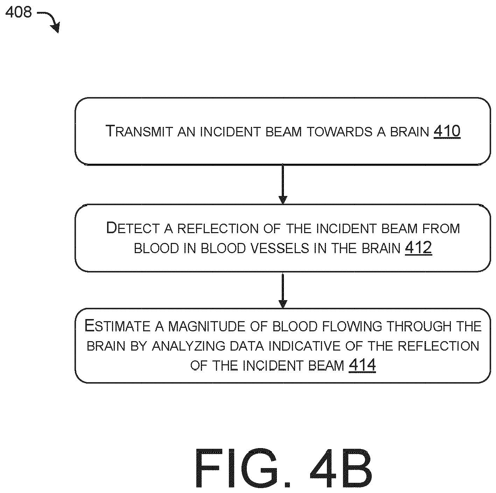

FIG. 4B illustrates an example process 408 for determining a magnitude of blood flowing through a brain of a subject. In various examples, the process 408 can be performed by an entity. The entity may include the flow monitor 120, the display device 132, the flow monitor 220, the display device 232, a medical device, a resuscitation system, an external flow monitor, a monitor-defibrillator, a mechanical chest compression device configured to administer chest compressions to the subject, a computing device, at least one processor, or any combination thereof. In some cases, the entity may include a housing and a strap coupled to the housing and configured to hold the external flow monitor on an external surface of a head of the subject.

At 410 the entity transmits an incident beam toward a brain of a subject. In some cases, the subject is an adult subject. The entity may include an emitter configured to output the incident beam. In some cases, the emitter may be disposed within the housing of the entity. The incident beam may include ultrasound or light. In some cases, the ultrasound may have a frequency in a range of 20 kilohertz (kHz) to 200 megahertz (MHz). For example, the ultrasound may have a frequency in a range of 100 kHz to 2 MHz. In some cases, the incident beam may include infrared light having, for example, a frequency in a range of 300 GHz - 430 THz and a wavelength in a range of 700 nanometers (nm) to 1 millimeter (mm). In particular embodiments, the incident beam may be transmitted toward blood vessels in the brain through an opening in the skull, such as an orbital cavity, a temple, a nose, or an ear canal of the subject. Blood vessels, for example, may be disposed in a circle of Willis of the subject.

At 412 the entity detects a reflection of the incident beam from blood in blood vessels in the brain of the subject. The entity may include a receiver configured to detect the reflection of the incident beam from blood vessels in the brain. For example, the receiver may be configured detect the reflection of the incident beam from blood, including blood cells and plasma in blood vessels, and/or a contrast agent disposed in the blood in the blood vessels. In some cases, the receiver may be disposed within the housing of the entity. In some cases, the receiver may be configured to generate an analog signal by detecting a reflection of the incident beam from a blood vessel in the brain of the subject. The entity may include an ADC configured to convert the analog signal into a digital signal. The entity may include one or more processors configured to analyze data. In some cases, the entity determines a volume of blood flowing in the blood vessel in the brain of the subject by analyzing the digital signal.

At 414 the entity, for example, may estimate a magnitude of blood flowing through the brain during an event by analyzing data indicative of the reflection of the incident beam. The event may include a chest compression, a cardiac cycle, or a ventilation cycle. The entity may determine the magnitude of the blood flowing through the brain, for example, by comparing a frequency of the reflection of the incident beam to a frequency of the incident beam. In some cases, the entity determines a volume of the blood flowing through the brain by integrating the velocity of the blood flowing in the blood vessel with respect to time.

The entity may perform various additional functions based on the magnitude, volume, and/or velocity of blood through the brain. The entity may output a signal indicative of the magnitude of blood flowing through the brain to a user, to a computing device, such as a mobile device, and/or to a medical device such as, a defibrillator, or a mechanical chest compression device. In some cases, the signal may be transmitted to the computing device and/or medical device. In some cases, the entity includes an output device configured to output the signal. In some cases, the output device may be a transceiver. In particular embodiments, the entity may determine that the magnitude of the blood flowing through the brain is below a threshold. The signal indicative of the magnitude of blood flowing through the brain may, for example, include instructions to initiate chest compressions, or change a position, frequency, timing, or depth of additional chest compressions. In particular embodiments, the entity determines that the volume and/or velocity of the blood flowing through the brain is below a threshold. The entity may output a signal indicative of the magnitude of blood flowing in the blood vessel which may, for example, include an instruction to initiate or change additional chest compressions administered to the subject. In some cases, the entity determines that the volume of the blood flowing through a blood vessel in the brain is below a threshold. In response to determining that the volume of blood flowing in the blood vessel in the brain is below the threshold, the entity may, for example, cause the transceiver to transmit, to the mechanical chest compression device, a signal instructing the mechanical chest compression device to change a position, frequency, timing, or depth of the chest compressions.

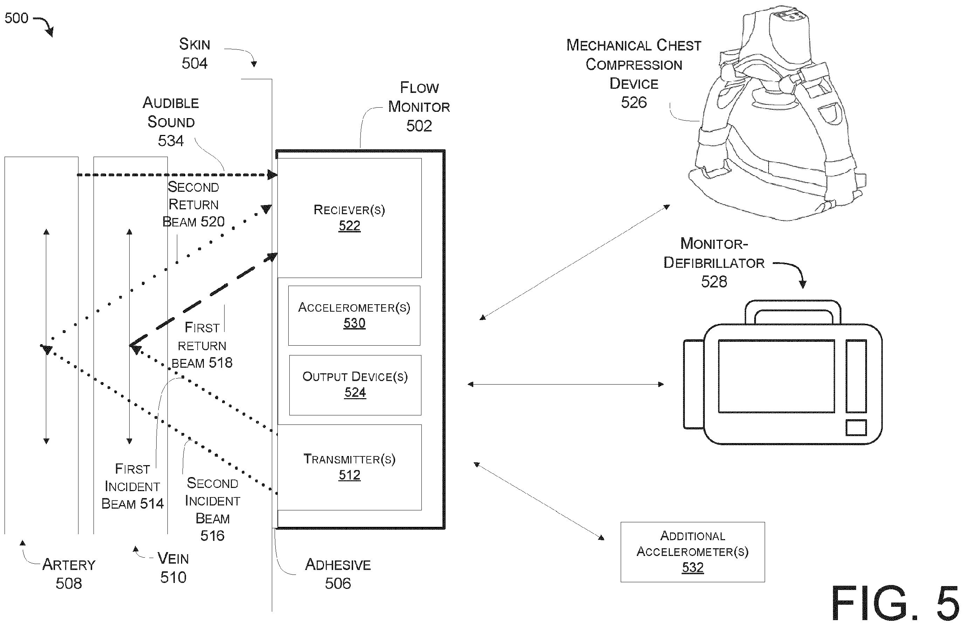

FIG. 5 illustrates an example environment 500 for monitoring blood flow through one or more blood vessels of a subject. A flow monitor 502 is adhered to skin 504 of the subject via an adhesive 506. In some examples, the flow monitor 502 is the flow monitor 120, the flow monitor 220, or the flow monitor 300. The flow monitor 502, for instance, is located outside of the body of the subject. In some implementations, the flow monitor 502 is held on the skin 504 by a strap, a buckle, a bandage, or some other fastener. For instance, the flow monitor 502 may be wrapped around an extremity of the subject.

In various implementations, an artery 508 and a vein 510 are disposed underneath the skin 504. The artery 508 and the vein 510 are part of the circulatory system of the subject. The circulatory system includes a fluid circuit of various blood vessels (including the artery 508 and the vein 510). The subject includes a heart that, when functioning, pumps blood through the blood vessels. In particular, the heart moves blood from lungs of the subject, where the blood can be oxygenated, to other portions of the subject's body, such as the brain, other organs, and the subject's extremities. Along the circulatory system, cells within the blood deliver oxygen to cells of the subject, thereby supporting cellular respiration. In various cases, the artery 508 carries oxygenated blood from the lungs of the subject and the vein 510 carries deoxygenated blood toward the lungs. Examples of the artery 508 include a carotid artery, a subclavian artery, a coronary artery, a brachial artery, an iliac artery, a radial artery, a femoral artery, or a pulmonary artery. Examples of the vein 510 include a jugular vein, an iliac vein, a subclavian vein, a cephalic vein, a brachial vein, a basilic vein, a hepatic vein, a radial vein, an ulnar vein, a digital vein, a brachiocephalic vein, a femoral vein, a saphenous vein, a venous arch, or a tibial vein. In some cases, the pulmonary artery carries deoxygenated blood.

According to various implementations of the present disclosure, the flow monitor 502 is configured to detect the flow of blood through the artery 508 and/or vein 510. In particular cases, the flow monitor 502 includes one or more transmitters 512 configured to output one or more incident beams toward the artery 508 and/or vein 510. In the example illustrated in FIG. 5, the incident beams include a first incident beam 514 and a second incident beam 516.

In various cases, the first incident beam 514 and the second incident beam 516 include waves that are transmitted through the skin 504. The waves, for example, can be instantiated as light and/or sound. In various cases, the first incident beam 514 and the second incident beam 516 include at least one of infrared, near-infrared, or visible light. For instance, the light may have a frequency in a range of 300 GHz - 530 THz and a wavelength in a range of 700 nanometers (nm) to 1 millimeter (mm). For instance, the transmitter(s) 512 include one or more light sources, such as light-emitting diodes (LEDs) or lasers. In some examples, the transmitter(s) 512 may include one or more mirrors configured to split the light output by the light source(s), such as for an interferometric analysis. In some cases, the receiver(s) 522 include one or more light sensors, such as at least one of a photodiode, a phototransistor, a photomultiplier tube, a charge-coupled device, a metal-semiconductor-metal photodetector, or a complementary metal oxide semiconductor photodetector.

According to various implementations, the waves include ultrasound. As used herein, the term "ultrasound," and its equivalents, can refer to mechanical waves (e.g., in the form of pressure waves) having a frequency in a range of 20 kilohertz (kHz) to 200 megahertz (MHz). Ultrasound, for example, is sound in a frequency that is greater than an upper detection limit of a human ear. In various instances, transmitter(s) 512 include one or more piezoelectric crystals (including, e.g., lead zirconate titanate (PZT), LiNbO₃ (LN), lead magnesium niobate-lead titanate (PMN-PT), or lead indium niobate- lead magnesium niobate-lead titanate (PIN-PMN-PT)). When an electrical current is induced through the piezoelectric crystal(s), the piezoelectric crystal(s) vibrate at a frequency that produces ultrasound. In some examples, the transmitter(s) 512 include one or more micro-electromechanical system (MEMS) devices. In some instances, the transmitter(s) 512 include one or more capacitive micromachined ultrasonic transducers (CMUTs) and/or piezoelectric micromachined ultrasonic transducers (PMUT). Any electrical to mechanical conversion system or material that operates at the ultrasound frequency range would be suitable.

According to various implementations, the flow monitor 502 includes one or more ultrasound transducers. As used herein, the terms "ultrasound transducer," "ultrasonic transducer," "transducer," and their equivalents, may refer to a device that generates or detects ultrasound. For instance, the ultrasound transducer(s) include the transmitter(s) 512 and/or the receiver(s) 522. In some cases, an ultrasound transducer includes a transducer element (e.g., a piezoelectric crystal, MEMS device, or another type of electrical-to-mechanical conversion device), a first electrode disposed on one side of the transducer element, and a second electrode disposed on another side of the transducer element. In various implementations, the ultrasound transducer is configured to produce ultrasound by inducing a current through or a voltage between the first and second electrodes. In some cases, the ultrasound transducer is configured to detect ultrasound by detecting a current through or voltage between the first and second electrodes that is induced when the ultrasound is received by the transducer element. In some cases, the ultrasound transducer is encased in a housing, which may be watertight. The ultrasound transducer, in some examples, further includes a matching layer that is disposed between the transducer element and a surface of the housing from which an incident beam of ultrasound is emitted. The matching layer includes a material having an acoustic impedance that is between the acoustic impedance of the transducer element and an acoustic lens (if one exists or between the transducer and skin). In some implementations, a gel layer is disposed between the housing of the ultrasound transducer and the skin 504 that further matches the impedance between the ultrasound transducer and the body, thereby preventing the first incident beam 514 and the second incident beam 516 from being reflected by an interface containing air between the skin 504 and the ultrasound transducer. In some cases, the adhesive 506 serves as the gel layer.

In some examples, the first incident beam 514 is reflected and/or scattered by blood in the vein 510, thereby generating a first return beam 518. Further, in some cases, the second incident beam 516 is reflected and/or scattered by blood in the artery 508, thereby generating a second return beam 520. The flow monitor 502 includes one or more receivers 522 configured to detect the first return beam 518 and/or the second return beam 520.

As illustrated, the artery 508 and the vein 510 are substantially parallel to the skin 504. In various implementations of the present disclosure, the transmitter(s) 512 emit the first incident beam 514 and/or the second incident beam 516 in a direction that is non-perpendicular and non-parallel to the surface of the flow monitor 502 that is adhered to the skin 504. That is, the transmitter(s) 512 emit the first incident beam 514 and/or the second incident beam 516 in an angled fashion. Thus, a component of the first incident beam 514 is parallel to the blood flow through the vein 510 and/or a component of the second incident beam 516 is parallel to the blood flow through the artery 508.

Various implementations of the transmitter(s) 512 that enable angled transmission of the first incident beam 514 and the second incident beam 516 are disclosed herein. In some implementations in which the transmitter(s) 512 include one or more transducer elements configured to emit ultrasound, an individual transducer element may have the shape of a cylinder (with a height of the cylinder being substantially parallel to the skin 504 and perpendicular to a direction of the artery 508 and/or vein 510) or a polygonal prism (with a height of the polygonal prism being substantially parallel to the skin 504 and perpendicular to a direction of the artery 508 and/or vein 510). In some cases, a piezoelectric crystal may be divided into sections defined by an angle perpendicular to the height of the cylinder, each section serving as a different transducer element configured to emit ultrasound. In some cases, the transmitter(s) 512 include multiple (e.g., planar) transducer elements that emit respective ultrasound beams that are phase networked together such that they collectively form the angled first incident beam 514 or the second incident beam 516. In some cases, the transmitter(s) 512 include a single transducer element including portions that are insensitive to ultrasound (e.g., non-piezoelectric portions, such as including a polymer) and portions that are sensitive to ultrasound (e.g., piezoelectric portions), which can cause the first incident beam 514 or the second incident beam 516 to be output as a grating lobe. The grating lobe may include components emitted at different angles, at least one of which may include the first incident beam 514 or the second incident beam 516 to be transmitted at an angle with respect to the artery 508 or vein 510.

In some implementations, the transmitter(s) 512 include a transducer element that emits ultrasound from a surface that is nonparallel to the skin 504. For example, the flow monitor 502, in some cases, includes a wedge-shaped spacer between the crystal and the skin 504. The spacer may be substantially acoustically transparent. In some examples, the wedge-shaped spacer includes a polymer or gel that is configured to perform impedance matching between the crystal and the skin 504. For instance, the spacer may include multiple layers arranged in steps that are configured to be in contact with the crystal and/or tilt the crystal with respect to the skin 504.

In some cases, the flow monitor 502 includes an acoustic "lens" that is disposed between a crystal emitting ultrasound and the skin 504. For example, the acoustic lens can be a spacer with a nonuniform acoustic impedance. Thus, the acoustic lens may bend the ultrasound emitted by the crystal before it is transmitted through the skin 504.

In some examples, the transmitter(s) 512 includes a crystal that is disposed inside of a needle that is disposed through the skin 504. In some implementations, the transmitter(s) 512 include an array of crystals configured to emit the first incident beam 514 and/or the second incident beam 516 through the skin 504. For example, the array may be arranged on a curved surface, such that individual crystals may point in different directions. In operation, the flow monitor 502 may automatically determine which incident beams whose return beams produce a greatest frequency and/or phase shift with respect to the incident beams, and may define those beams as the first incident beam 514 and/or the second incident beam 516.

In various implementations, the first return beam 518 may represent a frequency and/or phase shift with respect to the first incident beam 514 due to the Doppler effect. Similarly, because the component of the second incident beam 516 is parallel to the blood flow through the artery 508, the second return beam 520 may represent a frequency and/or phase shift with respect to the second incident beam 516 due to the Doppler effect. These shifts occur due to the Doppler effect and may be referred to as "Doppler shifts."

According to various implementations, the flow monitor 502 determines a velocity of the blood flow through the artery 508 and the vein 510 due to a difference between the frequencies of the first incident beam 514 and the first return beam 518, as well as a difference between the frequencies of the second incident beam 516 and the second return beam 520. The transmitter(s) 512, in some cases, operate in a continuous wave Doppler mode (also referred to as "CW Doppler"), and continuously transmit the first incident beam 514 and the second incident beam 516 during a monitoring period. The receiver(s) 522, for instance, continuously detect the first return beam 518 and the second return beam 520 during the monitoring period. For instance, the flow monitor 502 detects the blood velocity in the artery 508 and the blood velocity in the vein 510, in-real time, continuously or semi-continuously based on the frequency shifts between the first incident beam 514 and the first return beam 518 as well as between the second incident beam 516 and the second return beam 520.

In various implementations, the flow monitor 502 operates in a pulsed-wave Doppler mode (also referred to as "PW Doppler"). For instance, the flow monitor 502 causes the incident beam 516 to output the first incident beam 514 and/or the second incident beam 516 in pulses and detects the first return beam 518 and/or the second return beam 520 as return pulses. In various cases, a time delay between the output pulses and the return pulses is indicative of the depth of a structure from which the return pulses are reflected. In various implementations, the flow monitor 502 can determine the blood velocity in the artery 508 based on phase shifts between the pulses of the first incident beam 514 and the first return beam 518. Further, the flow monitor 502 can determine the blood velocity in the vein 510 based on phase shifts between the pulses of the second incident beam 516 and the second return beam 520.

In some cases, the flow monitor 502 detects the blood velocities at a sampling rate that corresponds to at least twice the component of the velocity range in the direction of the beam pointing angle of the first incident beam 514 and/or the second incident beam 516.

In some examples, the flow monitor 502 performs laser Doppler velocimetry in order to detect the blood velocity. In various cases, the flow monitor 502 includes one or more interferometric sensors. For example, the first incident beam 514 and the second incident beam 516 are light beams (e.g., coherent light beams) that are split (e.g., by one or more mirrors) prior to transmission through the skin 504. The flow monitor 502 may detect the blood velocities in the artery 108 and the vein 510 by comparing the first return beam 518 and the second return beam 520 to the split beams generated from the first incident beam 514 and the second incident beam 516. Techniques for interferometric detection of blood velocity can be found in, for example, R. D. Rader, C. M. Stevens and J. P. Meehan, "An Interferometric Blood Flow Measurement Technique - A Brief Analysis," in IEEE Transactions on Biomedical Engineering, vol. BME-21, no. 4, pp. 293-297, July 1974; Nagahara, et al., Method. Invest. Ophthalmol. Vis. Sci. 2011;52(1):87-92; and Robinson, et al., Sci Rep 13, 8803 (2023), each of which is incorporated by reference herein in its entirety.

In some cases, the flow monitor 502 images a portion of the subject that includes the artery 508 and the vein 510. In some cases, the flow monitor 502 generates an image using the first return beam 518 and the second return beam 520 using one or more sonographic techniques. In various implementations, the flow monitor 502 generates the image using multiple return beams including the first return beam 518 and the second return beam 520. The flow monitor 502, in some implementations, generates the multiple return beams by sweeping the first incident beam 514 and the second incident beam 516 across a section of the subject being imaged. For example, the flow monitor 502 may generate a real-time image of the subject that includes cross-sections of the artery 508 and vein 510, respectively. In some implementations, the flow monitor 502 automatically segments the cross-sections of the artery 508 and vein 510 in the real-time image. In some cases, the flow monitor 502 performs segmentation of a scrolling Doppler image (e.g., Doppler shift in a y-axis is swept in time along an x-axis) to segregate out the Doppler information of the vein 510 from the artery 508. Various types of segmentation techniques can be used, such as detection using histogram of oriented gradients (HOG) features, a scale-invariant feature transform (SIFT), Viola-Jones object detection framework, or You Only Look Once (YOLO). Once the cross-sections depicted in the image are identified using image segmentation, the flow monitor 502 can further classify the cross-sections using a support vector machine (SVM). In some cases, the flow monitor 502 uses one or more trained convolutional neural networks (CNNs) to segment and/or classify the cross-sections of the artery 508 and the vein 510 depicted in the image. In various cases, the flow monitor 502 may differentiate the cross-section corresponding to the artery 508 an the cross-section corresponding to the vein 510.

In particular implementations, the flow monitor 502 detects the velocity of the blood through the artery 508 and the velocity of the blood through the vein 510, simultaneously. For example, the first return beam 518 may be reflected from the artery 508 and the second return beam 520 may be reflected from the vein 510, so that the flow monitor 502 can detect the blood velocity of the artery 508 based on the first return beam 518 and may detect the blood velocity of the vein 510 based on the second return beam 520.

Simultaneously monitoring the blood velocity through the artery 508 and the vein 510 may enable certain evaluations of the subject. In some implementations, the flow monitor 502 detects a volumetric flow rate or net flow volume (e.g., during a time period) through at least a portion of the subject based on the blood velocity through the artery 508 and the vein 510. In some cases, the flow monitor 502 determines the volumetric flow rate through the artery 508 by integrating the velocity of the blood through the artery 508 across a cross-sectional area of the artery 508. The flow monitor 502 may determine the volumetric flow rate through the vein 510 by integrating the velocity of the blood through the vein 510 across a cross-sectional area of the vein 510. In various implementations, the flow monitor 502 is configured to detect a net flow volume that flows through the cross-section of the artery 508 during a time period (e.g., a cardiac cycle, a chest compression cycle, a portion of a chest compression cycle, or any combination thereof) by integrating the volumetric flow rate through the artery 508 over the time period. Similarly, the flow monitor 502 is configured to detect a net flow volume that flows through the cross-section of the vein 510 during the time period by integrating the volumetric flow rate through the vein 510 over the time period.

The artery 508, for instance, supplies oxygenated blood to the portion of the subject, and the vein 510 transports deoxygenated blood from the portion of the subject. In some examples, the flow monitor 502 is configured to detect a net flow volume of blood to a portion of the subject's body over time. In particular cases, the artery 508 is a carotid artery and the vein 510 is a jugular vein, and the flow monitor 502 is configured to detect a net flow volume of blood to the brain of the subject over time.

The flow monitor 502 may assess a condition of the subject and/or evaluate a treatment administered to the subject by analyzing blood flow parameters (e.g., velocity, volumetric flow rate, or net flow volume of blood) in the artery 508 and/or the vein 510. In some implementations, the flow monitor 502 identifies chest compressions performed on the subject based on the blood velocity through the artery 508 and/or the blood velocity through the vein 510. When (e.g., effective) chest compressions are performed on the subject, the compressions push blood through the artery 508 and the vein 510 in a direction that moves distally from the chest. Thus, chest compressions cause blood flowing through the artery 508 and blood flowing through the vein 510 to travel in parallel directions. Moreover, an individual chest compression causes the blood to flow in the artery 508 and vein 510 in a surge. Thus, in some cases, the flow monitor 502 detects that chest compressions are being performed on the subject by detecting that the blood through the artery 508 and the blood through the vein 510 is traveling in the same or parallel directions (e.g., in a direction pointing distally from the chest). For instance, the flow monitor 502 can detect whether chest compressions are undesirably moving blood in the same direction through the artery 508 and the vein 510.

In some cases, the flow monitor 502 provides feedback about the efficacy of the chest compressions based on the blood velocity through the artery 508 and the vein 510 and/or the net flow volume to the portion of the subject. For instance, if the flow monitor 502 detects less than a threshold blood velocity through the artery 508 or vein 510, or detects less than a threshold net flow volume to the portion of the subject (e.g., during a particular time period), the flow monitor 502 can output a feedback signal via one or more output devices 524. The output device(s) 524, for instance, include a display (e.g., a screen configured to visually output signals), a speaker (e.g., configured to audibly output signals), a haptic feedback device (e.g., configured to convey signals by vibrating or otherwise moving), a transceiver (e.g., configured to transmit signals to external devices), or any combination thereof. In some cases, the feedback signal is output to a rescuer (not illustrated) performing the chest compressions, and may cause the rescuer to adjust the depth of the chest compressions, adjust the frequency of the chest compressions, or adjust the position of the chest compressions relative to the subject's chest.

In some implementations, the feedback signal is transmitted to a mechanical chest compression device 526 that is performing the chest compressions. The mechanical chest compression device 526, in some cases, administers the chest compressions by moving a plunger up and down on the subject's chest. The feedback signal, for instance, causes the mechanical chest compression device 526 to adjust the depth of the chest compressions, adjust the frequency of the chest compressions, adjust the position of the plunger relative to the subject's chest, pause chest compressions, or initiate chest compressions. In some implementations, the mechanical chest compression device 526 transmits a signal to the flow monitor 502 that indicates the timing, frequency, position, or another chest compression parameter characterizing the chest compressions administered by the mechanical chest compression device 526. The flow monitor 502, in some cases, generates the feedback signal based on the chest compression parameter, such that the feedback signal is specific to the conditions reported by the mechanical chest compression device 526.

The placement of chest compressions, in particular, impacts the flow of blood through specific blood vessels through the subject's body. In some cases, the flow monitor 502 enables optimization of the position of the chest compressions based on the flow rate through the artery 508, the flow rate through the vein 510, the net flow volume through the portion of the subject's body, a current position of the chest compressions, an identity or position of the artery 508 in the subject's body, an identity or position of the vein 510 in the subject's body, or any combination thereof. For example, if the chest compressions are being administered to the right of an ideal position, the flow monitor 502 may detect a peak and/or mean flow rate of blood in the artery 508 that is below a threshold. In various implementations, the flow monitor 502 (or another computing device that is communicatively coupled to the flow monitor 502) generates a feedback signal including an instruction to apply the chest compressions one direction or another and/or closer to the ideal position.

According to various implementations, the flow monitor 502 can detect the presence of spontaneous circulation of the subject, in which the heart of the subject is spontaneously pumping a sufficient amount of blood through the body of the subject. In some cases, the flow monitor 502 detects a return of spontaneous circulation (ROSC). The flow monitor 502, for instance, can detect spontaneous circulation while the subject is receiving chest compressions. As noted previously, when the subject is receiving chest compressions, the chest compressions cause parallel or unidirectional blood flow in the artery 508 and the vein 510. In contrast, the pumping heart of the subject circulating blood through the subject's circulatory system causes antiparallel or bidirectional blood flow in the artery 508 and the vein 510. Thus, the flow monitor 502, in some cases, detects spontaneous circulation in response to detecting antiparallel or bidirectional blood flow in the artery 508 and vein 510.

In some cases, the flow monitor 502 detects spontaneous circulation using other techniques. In general, the heart is capable of pumping blood at a greater peak velocity than chest compressions. Therefore, the monitor 502 may detect spontaneous circulation by detecting that a blood velocity through the artery 508 or the vein 510 is greater than a threshold. Further, the blood velocity through the artery 508 or the vein 510 with respect to time during chest compressions is different than the blood velocity through the artery 508 or the vein 510 with respect to time during spontaneous circulation. According to some implementations, the flow monitor detects spontaneous circulation by determining that at least one frequency component of the volumetric flow rate over time through the artery 508 or vein 510 has changed. For instance, a shape or morphology of the volumetric flow rate over time can be indicative of spontaneous circulation.

Certain functionalities of the flow monitor 502 are enhanced by communications with other devices, such as the mechanical chest compression device 526 and a monitor-defibrillator 528. Collectively, any combination of the flow monitor 502, the mechanical chest compression device 526, and the monitor-defibrillator 528 may be a resuscitation system that is configured to resuscitate a single subject. For example, the flow monitor 502 is configured to modify and/or temporally gate measurements it performs based on communications from the mechanical chest compression device 526 and/or the monitor-defibrillator 528. In some cases, the flow monitor 502 receives a communication signal from the mechanical chest compression device 526 that indicates a frequency of chest compressions administered by the mechanical chest compression device 526, a timing of chest compressions administered by the mechanical chest compression device 526, or a time of a pause in the chest compressions administered by the mechanical chest compression device 526. In some cases, the flow monitor 502 is configured to remove a chest compression artifact from a blood velocity over time based on the communication signal from the mechanical chest compression device 526. For instance, the flow monitor 502 may remove an artifact from the blood velocity by applying a band reject filter centered around the chest compression frequency or by applying a comb filter that includes the chest compression frequency and one or more harmonics of the chest compression frequency. In some cases, the flow monitor 502 is configured to temporally gate a blood velocity measurement during a time window in which the mechanical chest compression device 526 has paused chest compressions or between chest compressions performed by the chest compression device 526. Using these techniques, in some cases, may enable the flow monitor 502 to more accurately identify spontaneous circulation of the subject by distinguishing between blood flow caused by the chest compressions and spontaneous blood flow induced by the heart of the subject.

In some cases, the monitor-defibrillator 528 transmits a communication signal to the flow monitor 502 indicating a time at which the monitor-defibrillator 528 is administering a treatment to the subject, such as an electrical shock or pace pulses. Because the electrical shock or pace pulses can interfere with the accuracy of other measurements performed by the flow monitor 502, the flow monitor 502 may gate the measurements at a time interval that omits the treatment to the subject. In some implementations, the flow monitor 502 includes sensitive electronics that could potentially be damaged when the treatment is administered to the subject. According to some examples, the flow monitor 502 includes a circuit with at least one switch that disconnects or otherwise shields the sensitive electronics during the treatment.

In some examples, the flow monitor 502 includes or is otherwise communicatively coupled to devices that include one or more sensors (not illustrated) configured to detect other physiological parameters. For example, the flow monitor 502 includes or is communicatively coupled with a sensor configured to detect an electrocardiogram (ECG), an oximetry sensor (e.g., a regional oximetry sensor, a pulse oximetry sensor, a cerebral oximetry sensor, or the like), or both.

In various implementations, the flow monitor 502 includes one or more accelerometers 530 configured to detect an acceleration of the flow monitor 502 and/or the skin 504 of the subject. The acceleration, for instance, is indicative of chest compressions administered to the subject or a pulse of the subject through the artery 508 or vein 510. Thus, in some cases, the flow monitor 502 detects whether the subject has a pulse based on the acceleration detected by the accelerometer(s) 530. At least one additional accelerometer 532 may further be disposed on another portion of the subject (e.g., the subject's chest) and may be configured to detect an acceleration indicative of the chest compressions. The flow monitor 502, in some implementations, removes a chest compression artifact of the acceleration detected by the accelerometer(s) 530 over time based on the acceleration detected by the additional accelerometer(s) 532 over time. In various implementations, the flow monitor 502 is configured to accurately detect spontaneous circulation or another condition of the subject based on the detected accelerations.

As used herein, the term "measurement" may refer to a blood velocity, a net blood flow volume, an ECG, an oxygenation of the subject's blood, an acceleration, or another physiological parameter of the subject. In some cases, the flow monitor 502 validates and/or temporally gates a first measurement in view of a second measurement. For example, if the ECG indicates that the heart of the subject is beating, but the blood velocity or net blood flow indicates that oxygenated blood is not circulating in the subject, then the flow monitor 502 may refrain from indicating that the subject has spontaneous circulation. In some cases, the flow monitor 502 may output a signal indicating that the subject has pulseless electrical activity (PEA).

In various cases, the flow monitor 502 is configured to detect blood flow in the brain of the subject. In some cases, the artery 508 is a part of the circle of Willis of the subject. If the subject is an adult, the skull of the subject may highly attenuate the first incident beam 514, the second incident beam 516, the first return beam 518, and the second return beam 520. For instance, the skull may have high attenuation with respect to ultrasound and/or light (e.g., at certain frequencies). Thus, in various examples, the flow monitor 502 is configured to direct the first incident beam 514, the second incident beam 516, the first return beam 518, and the second return beam 520 through a window in the skull. For instance, the flow monitor 502 is configured to monitor the artery 508 and/or vein 510 through a temple of the subject, an orbital socket of the subject, or an ear canal of the subject. In some cases, the flow monitor 502 is strapped to the head of the subject, such as in the form of an eyepatch.

In some cases, the operation of the flow monitor 502 is optimized in other ways to enable monitoring within the brain. For instance, if the first incident beam 514 and the second incident beam 516 include ultrasound, the ultrasound may have a frequency of less than 1 MHz. Although sub MHz ultrasound is incapable of generating high-resolution images, in some cases, the flow monitor 502 detects the flow through the artery 508 or vein 510 without generating an image of the artery 508 or vein 510. That is, sub MHz ultrasound is sufficient for Doppler flow detection in various implementations described herein.

Another way in which the flow monitor 502 is optimized for monitoring with the brain relates to the type of the artery 508 and/or vein 510 monitored by the flow monitor 502. Attenuation of an incident beam increases as it travels through an attenuating structure. Thus, in some cases, a distance between the flow monitor 502 (e.g., disposed on the skin 504 or eye) is less than a threshold distance.

In particular cases, it may be difficult to differentiate specific blood vessels in the brain (e.g., within the Circle of Willis) in which the first return beam 518 and/or the second return beam 520 are reflected and/or scattered. Further, it may be difficult to identify angles from which the blood vessels in the brain are disposed with respect to the flow monitor 502. Accordingly, it may be difficult to accurately quantify blood flow parameters of a blood vessel in the brain using techniques described herein. However, even detecting the presence or absence of movement of blood in the brain can provide helpful feedback in order to detect chest compression efficacy and/or spontaneous circulation. According to various implementations, the flow monitor 502 outputs an indication if the flow monitor 502 detects movement, or the absence of movement, or the relative difference of movement between the compression and decompression parts of CPR or the diastolic and systolic parts of a normal sinus rhythm of blood in the brain of the subject.

By detecting blood flow in the brain, the flow monitor 502 is configured to detect an efficacy of chest compressions being administered to the subject. Technologies that monitor blood flow in extremities can indirectly detect chest compression efficacy. However, because a primary purpose of chest compressions is to provide oxygenated blood to the brain of the subject, the flow monitor 502 is able to directly detect the efficacy of the chest compressions by monitoring blood flow in the brain. The flow monitor 502, for instance, can output a feedback signal (e.g., to the mechanical chest compression device 526 or to the user) indicating whether chest compressions are generating blood flow in the brain. In some cases, the feedback signal includes a metric indicative of the blood flow in the brain (e.g., a quantity that increases with an increase in a detected blood velocity or flow rate).

According to some examples, the receiver(s) 522 include one or more microphones configured to detect an audible sound 534 from the subject. For example, the flow monitor 502 may include the functionality of a sophisticated stethoscope. In some cases, the microphone(s) detect the audible sound 534 emitted by blood flowing through the artery 508 or vein 510. In some examples, the microphone(s) detect the audible sound 534 emitted by the heart of the subject. In various implementations, the audible sound 534 can be in an audible or inaudible range. The flow monitor 502, in various cases, detects the audible sound 534 at a sampling frequency, such as a sampling frequency of greater than 100 Hz, 1 kHz, or 10 kHz. In some implementations, the flow monitor 502 determines a condition of the subject based on the audible sound 534.

According to some cases, the output device(s) 524 include a speaker that outputs an audible signal indicative of the audible sound 534 detected by the flow monitor 502. For example, the output device(s) 524 output the sampled audible sound 534 into an audio headset of the user, or in an environment in which the user is present, at a volume that can be perceived by the user in an emergency scene. That is, the output device(s) 524 may amplify the audible sound 534. Some monitors output computer-generated sounds indicative of a heart rate of a subject, such as a "beep" sound whenever a QRS complex is detected in an ECG. According to some implementations, the audible sound 534 includes more diagnostic-relevant information that can be perceived by the user than a computerized "beep" sound. In some examples, a condition of the subject can be identified using the strength, velocity, or morphological characteristics of blood flow, but these features cannot necessarily be identified using audio output from a previous type of monitor. Furthermore, by outputting an amplified version of the audible sound 534 as an audible signal, the flow monitor 502 may convey potentially important information about the condition of the subject without visually outputting it on a display. The user, for example, may be monitoring other visual signals on the monitor-defibrillator 528, and thus the flow monitor 502 may enable cognitive offloading for the user.

In some implementations, the receiver(s) 522 include multiple microphones configured to detect audible sounds 534 from multiple locations on the subject's body. For instance, the microphones may detect the audible sound 534 from a right quadrant of the subject's chest and another audible sound 534 from a left quadrant of the subject's chest. The flow monitor 502, in some cases, analyzes the detected sounds for features indicative of a medical condition. For example, the flow monitor 502 detects the medical condition by detecting an anomaly in the detected sound. In some implementations, the flow monitor 502 selectively outputs a single one of the audible sounds based on an input signal received from the user. In some cases, the flow monitor 502 selectively outputs a single one of the audible sounds that is indicative of the medical condition. In various implementations, the output device(s) 524 are configured to output a signal indicating the medical condition.

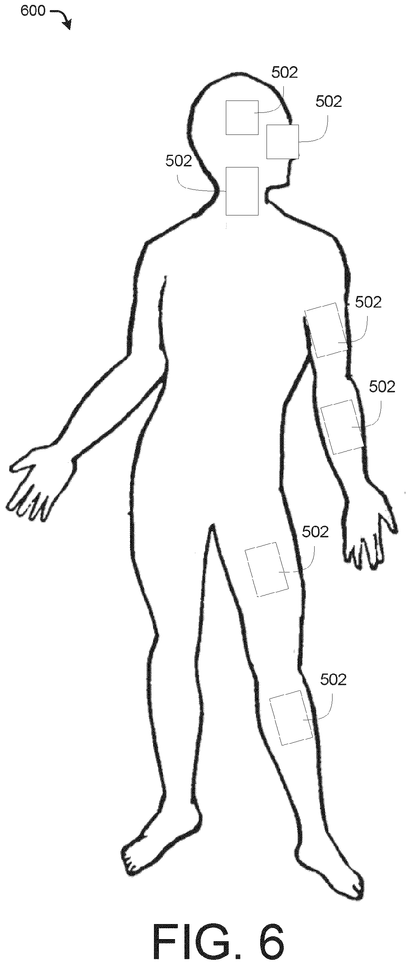

FIG. 6 illustrates various placements of a flow monitor 602 on the body of a subject 600. For example, the flow monitor 602 may be disposed on a temple, an orbital socket, an ear canal, a neck, an upper arm, a lower arm, an upper leg, or a lower leg of the subject 600. In some implementations, the flow monitor 602 includes multiple physical devices that are disposed on different parts of the body of the subject 600, simultaneously. In various implementations, the flow monitor 602 may be integrated into a cot or other support on which the subject 600 is disposed. In various cases, the flow monitor 602 may include at least one of the flow monitor 120, the flow monitor 220, the flow monitor 300, or the flow monitor 502.

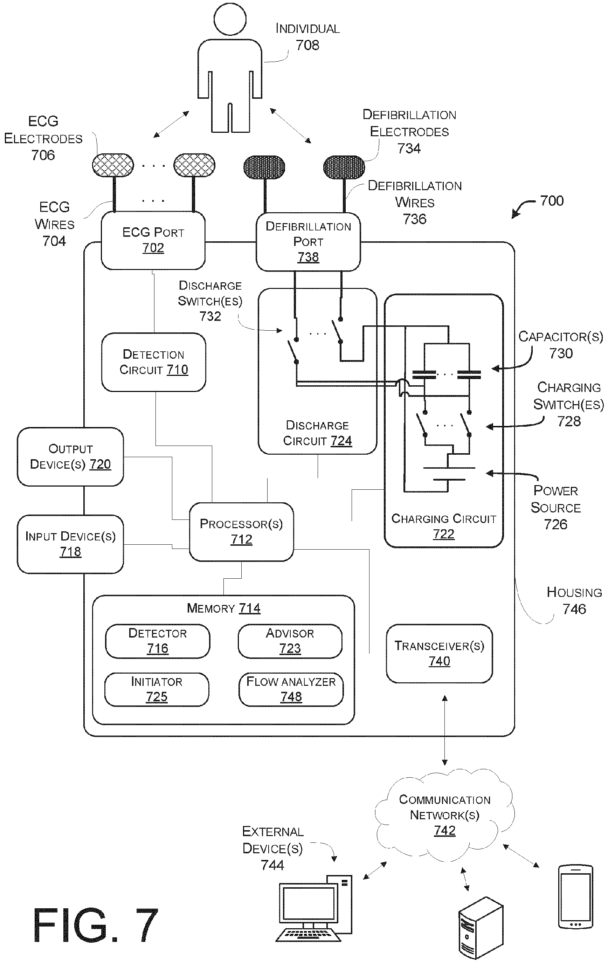

FIG. 7 illustrates an example of an external defibrillator 700 configured to perform various functions described herein. For example, the external defibrillator 7 is the monitor-defibrillator 528 described above with reference to FIG. 5.

The external defibrillator 700 includes an electrocardiogram (ECG) port 702 connected to multiple ECG wires 704. In some cases, the ECG wires 704 are removeable from the ECG port 702. For instance, the ECG wires 704 are plugged into the ECG port 702. The ECG wires 704 are connected to ECG electrodes 706, respectively. In various implementations, the ECG electrodes 706 are disposed on different locations on an individual 708. A detection circuit 710 is configured to detect relative voltages between the ECG electrodes 706. These voltages are indicative of the electrical activity of the heart of the individual 708.

In various implementations, the ECG electrodes 706 are in contact with the different locations on the skin of the individual 708. In some examples, a first one of the ECG electrodes 706 is placed on the skin between the heart and right arm of the individual 708, a second one of the ECG electrodes 706 is placed on the skin between the heart and left arm of the individual 708, and a third one of the ECG electrodes 706 is placed on the skin between the heart and a leg (either the left leg or the right leg) of the individual 708. In these examples, the detection circuit 710 is configured to measure the relative voltages between the first, second, and third ECG electrodes 706. Respective pairings of the ECG electrodes 706 are referred to as "leads," and the voltages between the pairs of ECG electrodes 706 are known as "lead voltages." In some examples, more than three ECG electrodes 706 are included, such that 5-lead or 12-lead ECG signals are detected by the detection circuit 710.

The detection circuit 710 includes at least one analog circuit, at least one digital circuit, or a combination thereof. The detection circuit 710 receives the analog electrical signals from the ECG electrodes 706, via the ECG port 702 and the ECG wires 704. In some cases, the detection circuit 710 includes one or more analog filters configured to filter noise and/or artifact from the electrical signals. The detection circuit 710 includes an analog-to-digital converter (ADC) in various examples. The detection circuit 710 generates a digital signal indicative of the analog electrical signals from the ECG electrodes 706. This digital signal can be referred to as an "ECG signal" or an "ECG."

In some cases, the detection circuit 710 further detects an electrical impedance between at least one pair of the ECG electrodes 706. For example, the detection circuit 710 includes, or otherwise controls, a power source that applies a known voltage (or current) across a pair of the ECG electrodes 706 and detects a resultant current (or voltage) between the pair of the ECG electrodes 706. The impedance is generated based on the applied signal (voltage or current) and the resultant signal (current or voltage). In various cases, the impedance corresponds to respiration of the individual 708, chest compressions performed on the individual 708, and other physiological states of the individual 708. In various examples, the detection circuit 710 includes one or more analog filters configured to filter noise and/or artifact from the resultant signal. The detection circuit 710 generates a digital signal indicative of the impedance using an ADC. This digital signal can be referred to as an "impedance signal" or an "impedance."

The detection circuit 710 provides the ECG signal and/or the impedance signal one or more processors 712 in the external defibrillator 700. In some implementations, the processor(s) 712 includes a central processing unit (CPU), a graphics processing unit (GPU), digital signal processing unit (DPU), other processing unit or component known in the art, or any combination thereof.

The processor(s) 712 is operably connected to memory 714. In various implementations, the memory 714 is volatile (such as random access memory (RAM)), non-volatile (such as read only memory (ROM), flash memory, etc.) or some combination of the two. The memory 714 stores instructions that, when executed by the processor(s) 712, causes the processor(s) 712 to perform various operations. In various examples, the memory 714 stores methods, threads, processes, applications, objects, modules, any other sort of executable instruction, or a combination thereof. In some cases, the memory 714 stores files, databases, or a combination thereof. In some examples, the memory 714 includes, but is not limited to, RAM, ROM, electrically erasable programmable read-only memory (EEPROM), flash memory, or any other memory technology. In some examples, the memory 714 includes one or more of CD-ROMs, digital versatile discs (DVDs), content-addressable memory (CAM), or other optical storage, magnetic cassettes, magnetic tape, magnetic disk storage or other magnetic storage devices, or any other medium which can be used to store the desired information and which can be accessed by the processor(s) 712 and/or the external defibrillator 700. In some cases, the memory 714 at least temporarily stores the ECG signal and/or the impedance signal.

In various examples, the memory 714 includes a detector 716, which causes the processor(s) 712 to determine, based on the ECG signal and/or the impedance signal, whether the individual 708 is exhibiting a particular heart rhythm. For instance, the processor(s) 712 determines whether the individual 708 is experiencing a shockable rhythm that is treatable by defibrillation. Examples of shockable rhythms include ventricular fibrillation (VF) and ventricular tachycardia (VT). In some examples, the processor(s) 712 determines whether any of a variety of different rhythms (e.g., asystole, sinus rhythm, atrial fibrillation (AF), etc.) are present in the ECG signal.

The processor(s) 712 is operably connected to one or more input devices 718 and one or more output devices 720. Collectively, the input device(s) 718 and the output device(s) 720 function as an interface between a user and the defibrillator 700. The input device(s) 718 is configured to receive an input from a user and includes at least one of a switch, a keypad, a cursor control, a touch-sensitive display, a voice input device (e.g., a microphone), a haptic feedback device (e.g., a gyroscope), or any combination thereof. The output device(s) 720 includes at least one of a display, a speaker, a haptic output device, a printer, a light indicator such as an LED, or any combination thereof. In various examples, the processor(s) 712 causes a display among the input device(s) 718 to visually output a waveform of the ECG signal and/or the impedance signal. In some implementations, the input device(s) 718 includes one or more touch sensors, the output device(s) 720 includes a display screen, and the touch sensor(s) are integrated with the display screen. Thus, in some cases, the external defibrillator 700 includes a touchscreen configured to receive user input signal(s) and visually output physiological parameters, such as the ECG signal and/or the impedance signal.

In some examples, the memory 714 includes an advisor 723, which, when executed by the processor(s) 712, causes the processor(s) 712 to generate advice and/or control the output device(s) 720 to output the advice to a user (e.g., a rescuer). In some examples, the processor(s) 712 provides, or causes the output device(s) 720 to provide, an instruction to perform CPR on the individual 708. In some cases, the processor(s) 712 evaluates, based on the ECG signal, the impedance signal, or other physiological parameters, CPR being performed on the individual 708 and causes the output device(s) 720 to provide feedback about the CPR in the instruction. According to some examples, the processor(s) 712, upon identifying that a shockable rhythm is present in the ECG signal, causes the output device(s) 720 to output an instruction and/or recommendation to administer a defibrillation shock to the individual 708.

The memory 714 also includes an initiator 725 which, when executed by the processor(s) 712, causes the processor(s) 712 to control other elements of the external defibrillator 700 in order to administer a defibrillation shock to the individual 708. In some examples, the processor(s) 712 executing the discharge circuit 724 selectively causes the administration of the defibrillation shock based on determining that the individual 708 is exhibiting the shockable rhythm and/or based on an input from a user (received, e.g., by the input device(s) 718. In some cases, the processor(s) 712 causes the defibrillation shock to be output at a particular time, which is determined by the processor(s) 712 based on the ECG signal and/or the impedance signal.

The processor(s) 712 is operably connected to a charging circuit 722 and a discharge circuit 724. In various implementations, the charging circuit 722 includes a power source 726, one or more charging switches 728, and one or more capacitors 730. The power source 726 includes, for instance, a battery. The processor(s) 712 initiates a defibrillation shock by causing the power source 726 to charge at least one capacitor among the capacitor(s) 730. For example, the processor(s) 712 activates at least one of the charging switch(es) 728 in the charging circuit 722 to complete a first circuit connecting the power source 726 and the capacitor to be charged. Then, the processor(s) 712 causes the discharge circuit 724 to discharge energy stored in the charged capacitor across a pair of defibrillation electrodes 734, which are in contact with the individual 708. For example, the processor(s) 712 deactivates the charging switch(es) 728 completing the first circuit between the capacitor(s) 730 and the power source 726, and activates one or more discharge switches 732 completing a second circuit connecting the charged capacitor 730 and at least a portion of the individual 708 disposed between defibrillation electrodes 734.

The energy is discharged from the defibrillation electrodes 734 in the form of a defibrillation shock. For example, the defibrillation electrodes 734 are connected to the skin of the individual 708 and located at positions on different sides of the heart of the individual 708, such that the defibrillation shock is applied across the heart of the individual 708. The defibrillation shock, in various examples, depolarizes a significant number of heart cells in a short amount of time. The defibrillation shock, for example, interrupts the propagation of the shockable rhythm (e.g., VF or VT) through the heart. In some examples, the defibrillation shock is 200 J or greater with a duration of about 0.015 seconds. In some cases, the defibrillation shock has a multiphasic (e.g., biphasic) waveform. The discharge switch(es) 732 are controlled by the processor(s) 712, for example. In various implementations, the defibrillation electrodes 734 are connected to defibrillation wires 736. The defibrillation wires 736 are connected to a defibrillation port 738, in implementations. According to various examples, the defibrillation wires 736 are removable from the defibrillation port 738. For example, the defibrillation wires 736 are plugged into the defibrillation port 738.

In various implementations, the processor(s) 712 is operably connected to one or more transceivers 740 that transmit and/or receive data over one or more communication networks 742. For example, the transceiver(s) 740 includes a network interface card (NIC), a network adapter, a local area network (LAN) adapter, or a physical, virtual, or logical address to connect to the various external devices and/or systems. In various examples, the transceiver(s) 740 includes any sort of wireless transceivers capable of engaging in wireless communication (e.g., radio frequency (RF) communication). For example, the communication network(s) 742 includes one or more wireless networks that include a 3^{rd} Generation Partnership Project (3GPP) network, such as a Long Term Evolution (LTE) radio access network (RAN) (e.g., over one or more LTE bands), a New Radio (NR) RAN (e.g., over one or more NR bands), or a combination thereof. In some cases, the transceiver(s) 740 includes other wireless modems, such as a modem for engaging in WI-FI^{®}, WIGIG^{®}, WIMAX^{®}, BLUETOOTH^{®}, or infrared communication over the communication network(s) 742.

The defibrillator 700 is configured to transmit and/or receive data (e.g., ECG data, impedance data, data indicative of one or more detected heart rhythms of the individual 708, data indicative of one or more defibrillation shocks administered to the individual 708, etc.) with one or more external devices 744 via the communication network(s) 742. The external devices 744 include, for instance, mobile devices (e.g., mobile phones, smart watches, etc.), Internet of Things (IoT) devices, medical devices (e.g., a flow monitor), computers (e.g., laptop devices, servers, etc.), or any other type of computing device configured to communicate over the communication network(s) 742. In some examples, the external device(s) 744 is located remotely from the defibrillator 700, such as at a remote clinical environment (e.g., a hospital). According to various implementations, the processor(s) 712 causes the transceiver(s) 740 to transmit data to the external device(s) 744. In some cases, the transceiver(s) 740 receives data from the external device(s) 744 and the transceiver(s) 740 provide the received data to the processor(s) 712 for further analysis.

In various implementations, the external defibrillator 700 also includes a housing 746 that at least partially encloses other elements of the external defibrillator 700. For example, the housing 746 encloses the detection circuit 710, the processor(s) 712, the memory 714, the charging circuit 722, the transceiver(s) 740, or any combination thereof. In some cases, the input device(s) 718 and output device(s) 720 extend from an interior space at least partially surrounded by the housing 746 through a wall of the housing 746. In various examples, the housing 746 acts as a barrier to moisture, electrical interference, and/or dust, thereby protecting various components in the external defibrillator 700 from damage.

In some implementations, the external defibrillator 700 is an automated external defibrillator (AED) operated by an untrained user (e.g., a bystander, layperson, etc.) and can be operated in an automatic mode. In automatic mode, the processor(s) 712 automatically identifies a rhythm in the ECG signal, makes a decision whether to administer a defibrillation shock, charges the capacitor(s) 730, discharges the capacitor(s) 730, or any combination thereof. In some cases, the processor(s) 712 controls the output device(s) 720 to output (e.g., display) a simplified user interface to the untrained user. For example, the processor(s) 712 refrains from causing the output device(s) 720 to display a waveform of the ECG signal and/or the impedance signal to the untrained user, in order to simplify operation of the external defibrillator 700.

In some examples, the external defibrillator 700 is a monitor-defibrillator utilized by a trained user (e.g., a clinician, an emergency responder, etc.) and can be operated in a manual mode or the automatic mode. When the external defibrillator 700 operates in manual mode, the processor(s) 712 cause the output device(s) 720 to display a variety of information that may be relevant to the trained user, such as waveforms indicating the ECG data and/or impedance data, notifications about detected heart rhythms, and the like.

In some implementations, the external device(s) 744 include a device including a sensor that is configured to detect blood flow in the body of the individual 708. The external device(s) 744, for instance, indicate the detected blood flow in a communication signal to the defibrillator 700. In some implementations, the memory 714 includes a flow analyzer 748 which, when executed by the processor(s) 712, causes the processor(s) 712 to perform various functions related to calculating flow parameters, detecting blood flow in and/or through the brain of the individual 708, and performing various actions based on the detected blood flow. For example, the flow analyzer 748 may cause the processor(s) 712 to perform various functions of flow monitoring devices described herein.

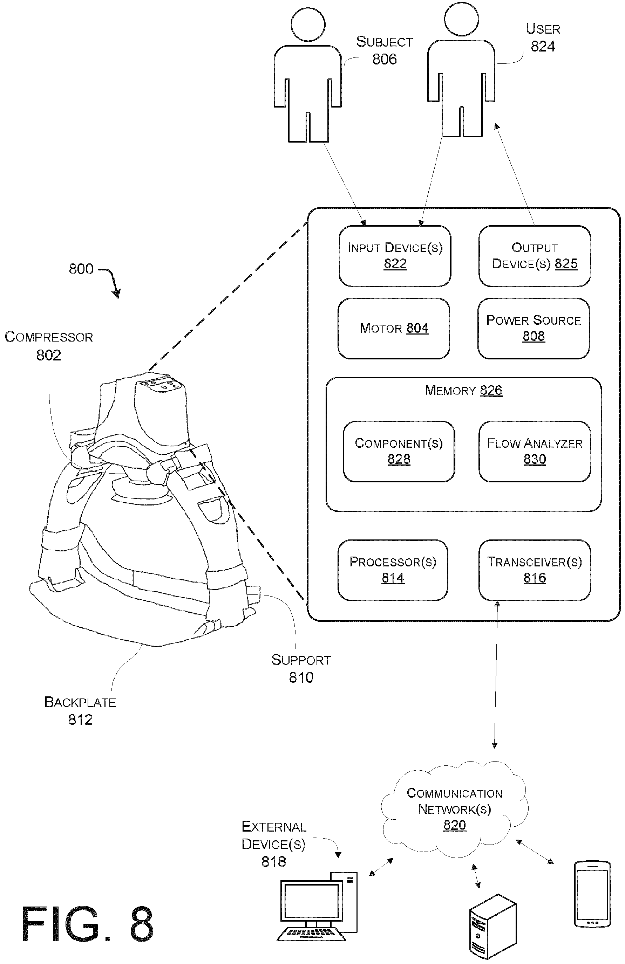

FIG. 8 illustrates a chest compression device 800 configured to perform various functions described herein. For example, the chest compression device 800 is the mechanical chest compression device 526 described with reference to FIG. 5.

In various implementations, the chest compression device 800 includes a compressor 802 that is operatively coupled to a motor 804. The compressor 802 physically administers a force to the chest of a subject 806 that compresses the chest of the subject 806. In some examples, the compressor 802 includes at least one piston that periodically moves between two positions (e.g., a compressed position and a release position) at a compression frequency. For example, when the piston is positioned on the chest of the subject 806, the piston compresses the chest when the piston is moved into the compressed position. A suction cup may be positioned on a tip of the piston, such that the suction cup contacts the chest of the subject 806 during operation. In various cases, the compressor 802 includes a band that periodically tightens to a first tension and loosens to a second tension at a compression frequency. For instance, when the band is disposed around the chest of the subject 806, the band compresses the chest when the band tightens.

The motor 804 is configured to convert electrical energy stored in a power source 808 into mechanical energy that moves and/or tightens the compressor 802, thereby causing the compressor 802 to administer the force to the chest of the subject 806. In various implementations, the power source 808 is portable. For instance, the power source 808 includes at least one rechargeable (e.g., lithium-ion) battery. In some cases, the power source 808 supplies electrical energy to one or more elements of the chest compression device 800 described herein.

In various cases, the chest compression device 800 includes a support 810 that is physically coupled to the compressor 802, such that the compressor 802 maintains a position relative to the subject 806 during operation. In some implementations, the support 810 is physically coupled to a backplate 812, cot, or other external structure with a fixed position relative to the subject 806. According to some cases, the support 810 is physically coupled to a portion of the subject 806, such as wrists of the subject 806.

The operation of the chest compression device 800 may be controlled by at least one processor 814. In various implementations, the motor 804 is communicatively coupled to the processor(s) 814. Specifically, the processor(s) 814 is configured to output a control signal to the motor 804 that causes the motor 804 to actuate or otherwise adjust the compressor 802. For instance, the motor 804 causes the compressor 802 to administer the compressions to the subject 806 based on the control signal. In some cases, the control signal indicates one or more treatment parameters of the compressions. Examples of treatment parameters include a frequency, timing, depth, force, position, velocity, and acceleration of the compressor 802 administering the compressions. According to various cases, the control signal causes the motor 804 to cease compressions, such as after ROSC has been detected.

In various implementations, the chest compression device 800 includes at least one transceiver 816 configured to communicate with at least one external device 818 over one or more communication networks 820. Any wired and/or wireless communication network described herein can be included in the communication network(s) 820 illustrated in FIG. 8. The external device(s) 818, for example, includes at least one of a flow monitor, a monitor-defibrillator, an AED, an ECMO device, a ventilation device, a subject monitor, a mobile phone, a server, or a computing device. In some implementations, the transceiver(s) 816 is configured to communicate with the external device(s) 818 by transmitting and/or receiving signals wirelessly. For example, the transceiver(s) 816 includes a NIC, a network adapter, a LAN adapter, or a physical, virtual, or logical address to connect to the various external devices and/or systems. In various examples, the transceiver(s) 816 includes any sort of wireless transceivers capable of engaging in wireless communication (e.g., RF communication). For example, the communication network(s) 820 includes one or more wireless networks that include a 3GPP network, such as an LTE RAN (e.g., over one or more LTE bands), an NR RAN (e.g., over one or more NR bands), or a combination thereof. In some cases, the transceiver(s) 816 includes other wireless modems, such as a modem for engaging in WI-FI^{®}, WIGIG^{®}, WIMAX^{®}, BLUETOOTH^{®}, or infrared communication over the communication network(s) 820. The signals, in various cases, encode data in the form of data packets, datagrams, or the like. In some cases, the signals are transmitted as compressions are being administered by the chest compression device 800 (e.g., for real-time feedback by the external device(s) 818), after compressions are administered by the chest compression device 800 (e.g., for post-event review at the external device 818), or a combination thereof.

In various cases, the processor(s) 814 generates the control signal based on data encoded in the signals received from the external device(s) 818. For instance, the signals include an instruction to initiate the compressions, and the processor(s) 814 instructs the motor 804 to begin actuating the compressor 802 in accordance with the signals.

In some cases, the chest compression device 800 includes at least one input device 822. In various examples, the input device(s) 822 is configured to receive an input signal from a user 824, who may be a rescuer treating the subject 806. Examples of the input device(s) 822 include, for instance, a switch, a keypad, a cursor control, a touch-sensitive display, a voice input device (e.g., a microphone), a haptic feedback device (e.g., a gyroscope), or any combination thereof. In various implementations, the processor(s) 814 generate the control signal based on the input signal. For instance, the processor(s) 814 generate the control signal to adjust a frequency of the compressions based on the chest compression device 800 detecting a selection by the user 824 of a user interface element displayed on a touchscreen or detecting the user 824 pressing a button integrated with an external housing of the chest compression device 800.

According to some examples, the input device(s) 822 include one or more sensors. The sensor(s), for example, is configured to detect a physiological parameter of the subject 806. In some implementations, the sensor(s) is configured to detect a state parameter of the chest compression device 800, such as a position of the compressor 802 with respect to the subject 806 or the backplate 812, a force administered by the compressor 802 on the subject 806, a force administered onto the backplate 812 by the body of the subject 806 during a compression, or the like. According to some implementations, the signals transmitted by the transceiver(s) 816 indicate the physiological parameter(s) and/or the state parameter(s).

The chest compression device 800 further includes at least one output device 825, in various implementations. Examples of the output device(s) 825 include, for instance, least one of a display (e.g., a projector, an LED screen, etc.), a speaker, a haptic output device, a printer, a light source such as an LED, or any combination thereof. In some implementations, the output device(s) 825 include a screen configured to display various parameters detected by and/or reported to the chest compression device 800, a charge level of the power source 808, a timer indicating a time since compressions were initiated or paused, and other relevant information.

The chest compression device 800 further includes memory 826. In various implementations, the memory 826 is volatile (such as RAM), non-volatile (such as ROM, flash memory, etc.) or some combination of the two. The memory 826 stores instructions that, when executed by the processor(s) 814, causes the processor(s) 814 to perform various operations. In various examples, the memory 826 stores methods, threads, processes, applications, objects, modules, any other sort of executable instruction, or a combination thereof. In some cases, the memory 826 stores files, databases, or a combination thereof. In some examples, the memory 826 includes, but is not limited to, RAM, ROM, 8PROM, flash memory, or any other memory technology. In some examples, the memory 826 includes one or more of CD-ROMs, DVDs, CAM, or other optical storage, magnetic cassettes, magnetic tape, magnetic disk storage or other magnetic storage devices, or any other medium which can be used to store the desired information. In various cases, the memory 826 stores instructions, programs, threads, objects, data, or any combination thereof, that cause the processor(s) 814 to perform various functions. In various cases, the memory 826 stores one or more parameters that are detected by the chest compression device 800 and/or reported to the chest compression device 800.

In implementations of the present disclosure, the memory 826 also stores one or more components 828. The component(s) 828 include programs, instructions, files, databases, models, or any other type of data that causes the device 800 to perform any of the functions described herein.

In some implementations, the external device(s) 818 include a device including a sensor that is configured to detect blood flow in the body of the subject 806. The external device(s) 818, for instance, indicate the detected blood flow in a communication signal to the mechanical chest compression device 800. In some implementations, the memory 826 includes a flow analyzer 830 which, when executed by the processor(s) 814, causes the processor(s) 814 to perform various functions related to calculating flow parameters, detecting blood flow in and/or through the brain of the subject 806, and performing various actions based on the detected blood flow. For example, the flow analyzer 830 may cause the processor(s) 814 to perform various functions of flow monitoring devices described herein.

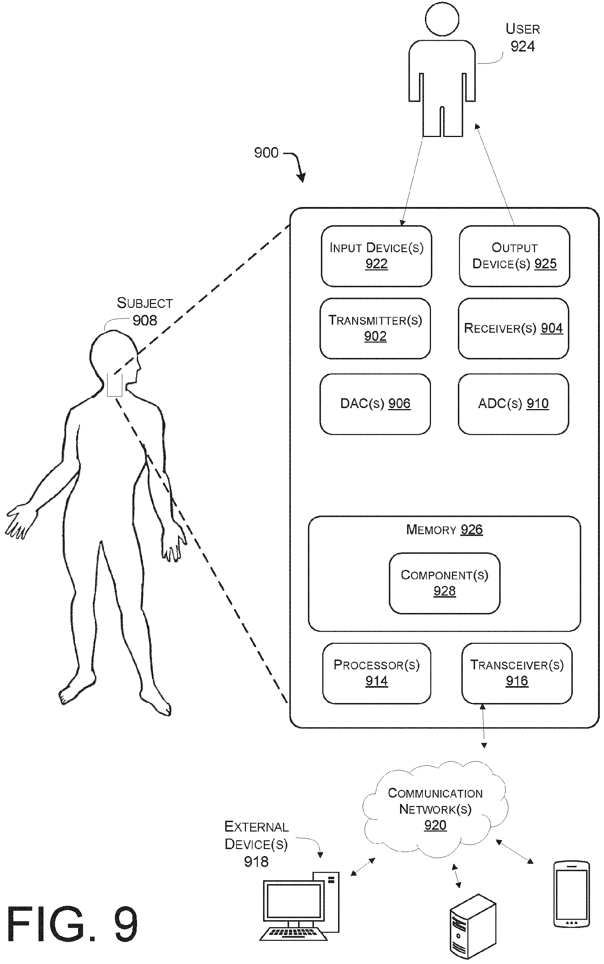

FIG. 9 illustrates a flow monitor 900 configured to perform various functions described herein. For example, the flow monitor 900 includes at least one of the flow monitor 120, the flow monitor 220, the flow monitor 300, the flow monitor 502, or the flow monitor 602.

In various implementations, the flow monitor 900 includes one or more transmitters 902, one or more receivers 904, one or more DACs 906, and one or more digital to analog converters 910. The transmitter(s) 902 is configured to emit an incident beam into a subject 908. The incident beam includes infrared light and/or ultrasound. For instance, the transmitter(s) 902 include one or more light sources (e.g., one or more LEDs) or one or more piezoelectric crystals, MEMS transducers, or other electro-mechanical conversion device or material. In some examples, the flow monitor 900 is positioned such that the incident beam is transmitted through a window in the skull of the subject 908, such as a temple, an orbital cavity, a nasal cavity, or an ear canal. In some cases, the transmitter(s) 902 emits one or more incident beams toward an artery supplying blood to a portion of the body of the subject 908 and a vein transporting blood out of the portion of the body of the subject 908.

In various implementations, the incident beam is reflected or otherwise scattered by blood in a blood vessel of the subject 908. The receiver(s) 904 is configured to detect within a return beam that includes signals from the reflection or scatter from the blood in the blood vessel. For example, the receiver(s) 904 include one or more light sensors or one or more piezoelectric crystals, MEMS transducers, or other electro-mechanical conversion device or material. In various cases, the flow monitor 900 includes one or more transceivers that embody the transmitter(s) 902 and receiver(s) 904.

The operation of the flow monitor 900 may be controlled by at least one processor 914. The processor(s) 914 are communicatively coupled to the DAC(s) 906 and the ADC(s) 910. The DAC(s) 906 is configured to convert digital signals output by the processor(s) 914 into analog signals, such as analog signals that induce the transmitter(s) 902 to emit the incident beam. The ADC(S) 910 is configured to convert analog signals generated by the receiver(s) 904 (e.g., induced by detecting the return beam) into digital signals that are received by the processor(s) 914. The processor(s) 914 is configured to control the transmitter(s) 902 and to analyze the signals detected by the receiver(s) 904. The processor(s) 914, for instance, analyze the digital signals from the ADC(s) 910, which are indicative of the return beam detected by the receiver(s) 904, in order to determine a flow velocity of the blood through the blood vessel. In some examples, the flow monitor 900 includes one or more switches (e.g., MOSFETs) connected to an array of transmitters within the transmitter(s) 902 that respectively connect the transmitter(s) 902 between one or more power rails (e.g., negative and/or positive power rails) and ground. The processor(s) 914, for instance, cause the switch(es) to connect each transmitter among the transmitter(s) 902 between ground and the power rail(s) in a periodic waveform, which causes the transmitter(s) 902 to selectively output incident beam(s) in accordance with the periodic waveform. In some cases, the waveform has a three half-cycle waveform and goes from a positive voltage, to a negative voltage, to a positive voltage, then to ground. In some cases, more than one positive power rail or more than one negative power rail is used for the same waveform.

In various implementations, the flow monitor 900 includes at least one transceiver 916 configured to communicate with at least one external device 918 over one or more communication networks 920. Any communication network described herein can be included in the communication network(s) 920 illustrated in FIG. 9. The external device(s) 918, for example, includes at least one of a mechanical chest compression device, a monitor-defibrillator, an AED, an ECMO device, a ventilation device, a subject monitor, a mobile phone, a server, or a computing device. In some implementations, the transceiver(s) 916 is configured to communicate with the external device(s) 918 by transmitting and/or receiving signals in a wired fashion and/or wirelessly. For example, the transceiver(s) 916 includes a NIC, a network adapter, a LAN adapter, or a physical, virtual, or logical address to connect to the various external devices and/or systems. In various examples, the transceiver(s) 916 includes any sort of wireless transceivers capable of engaging in wireless communication (e.g., RF communication). For example, the communication network(s) 920 includes one or more wireless networks that include a 3GPP network, such as an LTE RAN (e.g., over one or more LTE bands), an NR RAN (e.g., over one or more NR bands), or a combination thereof. In some cases, the transceiver(s) 916 includes other wireless modems, such as a modem for engaging in WI-FI^{®}, WIGIG^{®}, WIMAX^{®}, BLUETOOTH^{®}, or infrared communication over the communication network(s) 920. The signals, in various cases, encode data in the form of data packets, datagrams, or the like. In some cases, the signals are transmitted as compressions are being administered by the flow monitor 900 (e.g., for real-time feedback by the external device(s) 918), after compressions are administered by the flow monitor 900 (e.g., for post-event review at the external device 918), or a combination thereof.

In various cases, the processor(s) 914 generates the control signal based on data encoded in the signals received from the external device(s) 918. For instance, the signals include an instruction to initiate monitoring, and the processor(s) 914 outputs a control signal that causes the transmitter(s) 902 to emit the incident beam.

In some cases, the flow monitor 900 includes at least one input device 922. In various examples, the input device(s) 922 is configured to receive an input signal from a user 924, who may be a rescuer treating the subject 908. Examples of the input device(s) 922 include, for instance, a keypad, a cursor control, a touch-sensitive display, a voice input device (e.g., a microphone), a haptic feedback device (e.g., a gyroscope), or any combination thereof. In various implementations, the processor(s) 914 generate the control signal based on the input signal. For instance, the processor(s) 914 generate the control signal to adjust a frequency of the compressions based on the flow monitor 900 detecting a selection by the user 924 of a user interface element displayed on a touchscreen or detecting the user 924 pressing a button integrated with an external housing of the flow monitor 900.

According to some examples, the input device(s) 922 include one or more sensors. The sensor(s), for example, is configured to detect a physiological parameter of the subject 908. In some cases, the sensor(s) include one or more microphones, one or more accelerometers, one or more oximetry sensors, or one or more ECG sensors. The sensor(s), for instance, is configured to detect one or more physiological parameters. In some implementations, the sensor(s) is configured to detect a state parameter of the flow monitor 900, such as a position of the flow monitor 900 with respect to the subject 908 or the like. According to some implementations, the signals transmitted by the transceiver(s) 916 indicate the physiological parameter(s) and/or the state parameter(s).

The flow monitor 900 further includes at least one output device 925, in various implementations. Examples of the output device(s) 925 include, for instance, least one of a display (e.g., a projector, an LED screen, etc.), a speaker, a haptic output device, a printer, a light such as an LED, or any combination thereof. In some implementations, the output device(s) 925 include a screen configured to display various parameters detected by and/or reported to the flow monitor 900, a battery level of the flow monitor 900, and other relevant information.

The flow monitor 900 further includes memory 926. In various implementations, the memory 926 is volatile (such as RAM), non-volatile (such as ROM, flash memory, etc.) or some combination of the two. The memory 926 stores instructions that, when executed by the processor(s) 914, causes the processor(s) 914 to perform various operations. In various examples, the memory 926 stores methods, threads, processes, applications, objects, modules, any other sort of executable instruction, or a combination thereof. In some cases, the memory 926 stores files, databases, or a combination thereof. In some examples, the memory 926 includes, but is not limited to, RAM, ROM, 8PROM, flash memory, or any other memory technology. In some examples, the memory 926 includes one or more of CD-ROMs, DVDs, CAM, or other optical storage, magnetic cassettes, magnetic tape, magnetic disk storage or other magnetic storage devices, or any other medium which can be used to store the desired information. In various cases, the memory 926 stores instructions, programs, threads, objects, data, or any combination thereof, that cause the processor(s) 914 to perform various functions. In various cases, the memory 926 stores one or more parameters that are detected by the flow monitor 900 and/or reported to the flow monitor 900.

In implementations of the present disclosure, the memory 926 also stores one or more components 928. The component(s) 928 include programs, instructions, files, databases, models, or any other type of data that causes the flow monitor 900 to perform any of the functions described herein.

### EXAMPLE CLAUSES

1. A flow monitor, including: a first emitter configured to output a first incident beam; a first receiver configured to detect a reflection of the first incident beam from blood flowing through a carotid artery of a subject; a second emitter configured to output a second incident beam; a second receiver configured to detect a reflection of the second incident beam from blood flowing through a jugular vein of the subject; a display; and a processor configured to: determine a volume of blood flowing toward a brain of the subject by analyzing the reflection of the first incident beam; determine a volume of blood flowing from the brain of the subject by analyzing the reflection of the second incident beam; estimate a net flow of blood through a brain of the subject by determining an average of the volume of blood flowing from the brain of the subject and the volume of blood flowing toward the brain of the subject; and cause the display to visually output an indication of the net flow of blood through the brain.
2. The flow monitor of clause 1, wherein the subject is receiving chest compressions and the processor is further configured to: determine that the net flow of blood through the brain is below a threshold; and in response to determining that the net flow of blood through the brain is below the threshold, output an instruction to change a placement, a frequency, a depth, or timing of the chest compressions.
3. The flow monitor of clause 1 or 2, wherein the flow monitor further includes: a housing; and an adhesive disposed on the housing and configured to attach the flow monitor to a neck of the subject.
4. A method, including: determining, during an event, a volume of blood flowing through a carotid artery of a subject toward a brain of the subject, the event including a chest compression or a cardiac cycle; determining, during the event, a volume of blood flowing through a jugular vein of the subject away from the brain of the subject; estimating a net flow by averaging the volume of blood flowing through the carotid artery and the volume of blood flowing through the jugular vein; and outputting a signal indicative of the net flow of blood through the brain of the subject.
5. The method of clause 4, wherein determining, during the event, the volume of blood flowing through the carotid artery of the subject toward the brain of the subject includes: identifying a velocity of the blood flowing through the carotid artery; and integrating the velocity of the blood flowing through the carotid artery with respect to time, and wherein determining, during the event, the volume of blood flowing through the jugular vein of the subject away from the brain of the subject includes: identifying a velocity of the blood flowing through the jugular vein; and integrating the velocity of the blood flowing through the jugular vein with respect to time.
6. The method of clause 5, wherein identifying the velocity of the blood flowing through the carotid artery includes: detecting a reflection of a first incident beam from the blood flowing through the carotid artery; identifying a Doppler shift of the reflection of the first incident beam with respect to the first incident beam; and determining the velocity of the blood by analyzing the Doppler shift of the reflection of the first incident beam with respect to the first incident beam, and wherein identifying the velocity of the blood flowing through the jugular vein includes: detecting a reflection of a second incident beam from the blood flowing through the jugular vein; identifying a Doppler shift of the reflection of the second incident beam with respect to the second incident beam; and determining the velocity of the blood by analyzing the Doppler shift of the reflection of the second incident beam with respect to the second incident beam.
7. The method of clause 6, wherein the first incident beam and the second incident beam include ultrasound or light.
8. The method of any of clauses 4 to 7, wherein the event includes the chest compression, and wherein the method further includes: determining that the blood flows through the jugular vein and toward the brain of the subject when the subject is receiving the chest compression.
9. The method of clause 8, further including: determining an efficacy of the chest compression by comparing the net flow of blood through the brain of the subject to a threshold, and wherein the signal indicative of the net flow of blood through the brain of the subject is further indicative of the efficacy of the chest compression.
10. The method of clause 9, wherein determining the efficacy of the chest compression includes determining that the net flow of blood through the brain of the subject is below the threshold, and wherein the signal indicative of the net flow of blood through the brain of the subject includes an instruction to change a position, frequency, timing, or depth of an additional chest compression.
11. The method of any of clauses 4 to 10, wherein the signal indicative of the net flow of blood through the brain of the subject is output to a user.
12. The method of any of clauses 4 to 11, wherein the signal indicative of the net flow of blood through the brain of the subject is output to a defibrillator or to a mechanical chest compression device.
13. A flow monitor, including: a first sensor configured to detect, during an event, blood flowing through a carotid artery of a subject toward a brain of the subject, the event including a chest compression or a cardiac cycle; a second sensor configured to detect, during the event, blood flowing through a jugular vein of the subject away from the brain of the subject; an output device; and a processor configured to: determine a volume of the blood flowing through the carotid artery during the event; determine a volume of the blood flowing through the jugular vein during the event; estimate a net flow of blood through the brain of the subject by averaging the volume of blood flowing through the carotid artery and the volume of blood flowing through the jugular vein; and cause the output device to output a signal indicative of the net flow of blood through the brain of the subject.
14. The flow monitor of clause 13, wherein the processor is configured to determine the volume of blood flowing through the carotid artery of the subject toward the brain of the subject during the event by: identifying a velocity of the blood flowing through the carotid artery; and integrating the velocity of the blood flowing through the carotid artery with respect to time, and wherein the processor is configured to determine the volume of blood flowing through the jugular vein of the subject away from the brain of the subject during the event by: identifying a velocity of the blood flowing through the jugular vein; and integrating the velocity of the blood flowing through the jugular vein with respect to time.
15. The flow monitor of clause 14, wherein the first sensor is configured to detect, during the event, the blood flowing through the carotid artery by detecting a reflection of a first incident beam from the blood flowing through the carotid artery, and wherein the second sensor is configured to detect, during the event, the blood flowing through the jugular vein by detecting a reflection of a second incident beam from the blood flowing through the jugular vein.
16. The flow monitor of clause 15, wherein the first incident beam is reflected from a contrast agent disposed in the blood flowing through the carotid artery.
17. The flow monitor of clause 15 or 16, wherein the processor is further configured to identify a velocity of the blood flowing through the carotid artery by: identifying a Doppler shift of the reflection of the first incident beam with respect to the first incident beam; and determining the velocity of the blood by analyzing the Doppler shift of the reflection of the first incident beam with respect to the first incident beam, and wherein the processor is further configured to identify a velocity of the blood flowing through the jugular vein by: identifying a Doppler shift of the reflection of the second incident beam with respect to the second incident beam; and determining the velocity of the blood by analyzing the Doppler shift of the reflection of the second incident beam with respect to the second incident beam.
18. The flow monitor of any of clauses 15 to 17, wherein the first incident beam and the second incident beam include ultrasound or light.
19. The flow monitor of any of clauses 15 to 18, wherein the event includes the chest compression, and wherein the processor is further configured to: determine that the blood flows through the jugular vein toward the brain of the subject when the subject is receiving the chest compression.
20. The flow monitor of clause 19, wherein the processor is further configured to: determine an efficacy of the chest compression by comparing the net flow of blood through the brain of the subject to a threshold, and wherein the signal indicative of the net flow of blood through the brain of the subject is further indicative of the efficacy of the chest compression.
21. The flow monitor of clause 20, wherein the processor is configured to determine the efficacy of the chest compression by: determining that the net flow of blood through the brain of the subject is below the threshold, and wherein the signal indicative of the net flow of blood through the brain of the subject includes an instruction to change a position, frequency, timing, or depth of the chest compressions.
22. The flow monitor of any of clauses 13 to 21, wherein the output device is configured to output, to a user, the signal indicative of the net flow of blood through the brain of the subject.
23. The flow monitor of any of clauses 13 to 22, wherein the output device includes a transceiver, and wherein transceiver is configured to transmit the signal indicative of the net flow of blood through the brain of the subject to a defibrillator or to a mechanical chest compression device.
24. A resuscitation system, including: a mechanical chest compression device configured to administer chest compressions to a subject; and an external flow monitor including: a housing; a strap coupled to the housing and configured to hold the external flow monitor on an external surface of a head of the subject; an emitter disposed in the housing and configured to output an ultrasound beam toward the subject; a receiver disposed in the housing and configured to detect a reflection of the ultrasound beam from blood vessels in a brain of the subject; a transceiver; a processor configured to: determine a magnitude of blood flowing in the blood vessels in the brain of the subject by analyzing the reflection of the ultrasound beam from the blood vessel in the brain of the subject; determine that the magnitude of blood flowing in the blood vessels in the brain is below a threshold; and in response to determining that the magnitude of blood flowing in the blood vessels in the brain is below the threshold, cause the transceiver to transmit, to the mechanical chest compression device, a signal instructing the mechanical chest compression device to change a position, frequency, timing, or depth of the chest compressions.
25. The resuscitation system of clause 24, wherein the emitter is configured to output the ultrasound beam through an orbital cavity, a temple, or an ear canal of the subject, and wherein the blood vessels are located in a circle of Willis of the subject.
26. The resuscitation system of clause 24 or 25, wherein the ultrasound beam has a frequency in a range of about 100 kHz to about 2 MHz.
27. An external flow monitor, including: an emitter configured to output an incident beam toward a brain of an adult subject; a receiver configured to detect a reflection of the incident beam from blood vessels in the brain; a processor configured to: determine, by analyzing data indicative of the reflection of the incident beam, a magnitude of blood flowing through the brain during an event, the event including a cardiac cycle or chest compression; and output a signal indicative of the magnitude of blood flowing in the brain.
28. The external flow monitor of clause 27, wherein the incident beam includes ultrasound having a frequency in a range of about 100 kHz to about 2 MHz.
29. The external flow monitor of clause 27 or 28, wherein the incident beam includes infrared light.
30. The external flow monitor of any of clauses 27 to 29, wherein the emitter is configured to output the incident beam through an orbital cavity, a temple, or an ear canal of the subject.
31. The external flow monitor of any of clauses 27 to 30, wherein the processor is configured to determine the magnitude of the blood flowing in the brain by comparing a frequency of the reflection of the incident beam to a frequency of the incident beam.
32. The external flow monitor of any of clauses 27 to 31, wherein the receiver is configured to detect a reflection of the incident beam from a contrast agent disposed in the blood in the blood vessel.
33. The external flow monitor of any of clauses 27 to 32, further including: a transceiver, wherein the processor is configured to output the signal by causing the transceiver to transmit the signal to a medical device or a mobile device.
34. The external flow monitor of any of clauses 27 to 33, further including: an output device, wherein the processor is configured to output the signal by causing the output device to output the signal to a user.
35. The external flow monitor of any of clauses 27 to 34, wherein the processor is further configured to: determine a volume of the blood flowing through the brain by integrating a velocity of the blood flowing in the blood vessel with respect to time; and determine that the volume of the blood flowing through the brain is below a threshold, wherein the signal indicative of the magnitude of blood flowing in the blood vessel includes an instruction to initiate or change additional chest compressions administered to the subject.
36. A method, including: transmitting an incident beam toward a brain of a subject; detecting a reflection of the incident beam from blood in blood vessels in the brain of the subject; estimating a magnitude of blood flowing through the brain by analyzing data indicative of the reflection of the incident beam; and outputting a signal indicative of the magnitude of blood flowing through the brain.
37. The method of clause 36, wherein the incident beam includes ultrasound having a frequency in a range of about 100 kHz to about 2 MHz.
38. The method of clause 36 or 37, wherein the incident beam includes infrared light.
39. The method of any of clauses 36 to 38, wherein transmitting the incident beam toward the blood vessel in the brain of the subject includes transmitting the incident beam through an orbital cavity, a temple, or an ear canal of the subject.
40. The method of any of clauses 36 to 39, wherein determining the magnitude of the blood flowing through the brain includes comparing a frequency of the reflection of the incident beam to a frequency of the incident beam.
41. The method of any of clauses 36 to 40, wherein outputting the signal indicative of the magnitude of blood flowing through the brain includes transmitting the signal to a medical device or to a mobile device.
42. The method of any of clauses 36 to 41, wherein outputting the signal indicative of the magnitude of blood flowing through the brain includes causing an output device to output the signal to a user.
43. The method of any of clauses 36 to 42, further including: determining that the magnitude of the blood flowing through the brain is below a threshold, wherein the signal indicative of the magnitude of blood flowing through the brain includes an instruction to initiate or change chest compressions administered to the subject.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be used for realizing implementations of the disclosure in diverse forms thereof.

As will be understood by one of ordinary skill in the art, each implementation disclosed herein can comprise, consist essentially of or consist of its particular stated element, step, or component. Thus, the terms "include" or "including" should be interpreted to recite: "comprise, consist of, or consist essentially of." The transition term "comprise" or "comprises" means has, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the implementation to the specified elements, steps, ingredients or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11 % of the stated value; ±10% of the stated value; ±9% of the stated value; ±8% of the stated value; ±7% of the stated value; ±6% of the stated value; ±5% of the stated value; t4% of the stated value; t3% of the stated value; ±2% of the stated value; or ±1 % of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any non-claimed element essential to the practice of implementations of the disclosure.

Groupings of alternative elements or implementations disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain implementations are described herein, including the best mode known to the inventors for carrying out implementations of the disclosure. Of course, variations on these described implementations will become apparent to those of ordinary skill in the art upon reading the foregoing description. The inventors expect skilled artisans to employ such variations as appropriate, and the inventors intend for implementations to be practiced otherwise than specifically described herein. Accordingly, the scope of this disclosure includes all modifications and equivalents of the subject matter recited in the claims appended hereto as permitted by applicable law. Moreover, any combination of the above-described elements in all possible variations thereof is encompassed by implementations of the disclosure unless otherwise indicated herein or otherwise clearly contradicted by context.

## Claims

1. An external flow monitor, comprising:
an emitter configured to output an incident beam toward a brain of an adult subject;
a receiver configured to detect a reflection of the incident beam from blood vessels in the brain;
a processor configured to:
determine, by analyzing data indicative of the reflection of the incident beam, a magnitude of blood flowing through the brain during an event, the event comprising a cardiac cycle or chest compression; and
output a signal indicative of the magnitude of blood flowing in the brain.

2. The external flow monitor of claim 1, wherein the incident beam comprises ultrasound having a frequency in a range of 100 kHz to 2 MHz.

3. The external flow monitor of claim 1 or 2, wherein the incident beam comprises infrared light.

4. The external flow monitor of claim 1, 2 or 3, wherein the emitter is configured to output the incident beam through an orbital cavity, a temple, or an ear canal of the subject.

5. The external flow monitor of any of claims 1-4, wherein the processor is configured to determine the magnitude of the blood flowing in the brain by comparing a frequency of the reflection of the incident beam to a frequency of the incident beam.

6. The external flow monitor of any of claims 1-5, wherein the receiver is configured to detect a reflection of the incident beam from a contrast agent in the blood in the blood vessel.

7. The external flow monitor of any of claims 1-6, further comprising:
a transceiver,
wherein the processor is configured to output the signal by causing the transceiver to transmit the signal to a medical device or a mobile device.

8. The external flow monitor of any of claims 1-7, further comprising:
an output device,
wherein the processor is configured to output the signal by causing the output device to output the signal to a user.

9. The external flow monitor of any of claims 1-8, wherein the processor is further configured to:
determine a volume of the blood flowing through the brain by integrating a velocity of the blood flowing in the blood vessel with respect to time; and
determine that the volume of the blood flowing through the brain is below a threshold,
wherein the signal indicative of the magnitude of blood flowing in the blood vessel comprises an instruction to initiate or change additional chest compressions administered to the subject.

10. The external flow monitor of any of claims 1-9, further comprising a housing;
wherein the emitter is disposed in the housing;
wherein the receiver is disposed in the housing;

11. The external flow monitor of claim 10, comprising a strap coupled to the housing and configured to hold the external flow monitor on an external surface of a head of the subject.

12. A resuscitation system, comprising:
a mechanical chest compression device configured to administer chest compressions to a subject; and
the external flow monitor of claim 7, or any of claims 8-11 as far as dependent from claim 7;
wherein the processor is further configured to, on the basis of the determined magnitude of blood flowing in the blood vessels in the brain of the subject, cause the transceiver to transmit, to the mechanical chest compression device, a signal instructing the mechanical chest compression device to initiate chest compressions, and/or to change a position, frequency, timing, or depth of the chest compressions.

13. The resuscitation system of claim 12, wherein the processor is further configured to:
determine that the magnitude of blood flowing in the blood vessels in the brain is below a threshold; and
in response to determining that the magnitude of blood flowing in the blood vessels in the brain is below the threshold, cause the transceiver to transmit, to the mechanical chest compression device, a signal instructing the mechanical chest compression device to change a position, frequency, timing, or depth of the chest compressions.

14. A method, comprising:
transmitting an incident beam toward a brain of a subject;
detecting a reflection of the incident beam from blood in blood vessels in the brain of the subject;
estimating a magnitude of blood flowing through the brain by analyzing data indicative of the reflection of the incident beam; and
outputting a signal indicative of the magnitude of blood flowing through the brain.

15. The method of claim 14, further comprising:
determining that the magnitude of the blood flowing through the brain is below a threshold,
wherein the signal indicative of the magnitude of blood flowing through the brain comprises an instruction to initiate or change chest compressions administered to the subject.
